(19) **Europäisches Patentamt / European Patent Office / Office européen des brevets**

(11) **EP 3 727 418 B1**

(12) **EUROPEAN PATENT SPECIFICATION**

(45) Date of publication and mention of the grant of the patent:
**08.04.2026 Bulletin 2026/15**

(21) Application number: **18893009.3**

(22) Date of filing: **19.12.2018**

(51) International Patent Classification (IPC):
**A61K 9/16** *(2006.01)*       **A61K 38/17** *(2006.01)*
**A61K 9/00** *(2006.01)*       **C07K 14/785** *(2006.01)*

(52) Cooperative Patent Classification (CPC):
**A61K 9/0082; A61K 9/1617; A61K 38/395; C07K 14/785**

(86) International application number:
**PCT/US2018/066424**

(87) International publication number:
**WO 2019/126283 (27.06.2019 Gazette 2019/26)**

(54) **SURFACTANT FORMULATIONS FOR INHALATION**

TENSIDFORMULIERUNGEN ZUR INHALATION

FORMULATIONS DE TENSIOACTIFS INHALABLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **21.12.2017 US 201762609275 P**

(43) Date of publication of application:
**28.10.2020 Bulletin 2020/44**

(73) Proprietor: **Merz Pharmaceuticals, LLC Raleigh, NC 27615 (US)**

(72) Inventors:
- **LIPP, Michael, M.**
  **Framingham, MA 01710 (US)**
- **CHAN, Holly**
  **Braintree, MA 02148 (US)**

(74) Representative: **Lee, Nicholas John et al Kilburn & Strode LLP Lacon London 84 Theobalds Road London WC1X 8NL (GB)**

(56) References cited:
| | |
|---|---|
| WO-A1-01/58423 | WO-A1-2009/026434 |
| WO-A1-2012/030664 | WO-A1-2017/223502 |
| WO-A1-97/26863 | WO-A2-01/13891 |
| WO-A2-01/13893 | US-A1- 2004 042 970 |
| US-A1- 2008 226 730 | US-A1- 2016 317 503 |

- KAIALY WASEEM ED - BLANCO-PRIETO MARIA J ET AL: "A review of factors affecting electrostatic charging of pharmaceuticals and adhesive mixtures for inhalation", INTERNATIONAL JOURNAL OF PHARMACEUTICS, ELSEVIER, NL, vol. 503, no. 1, 2 February 2016 (2016-02-02), pages 262 - 276, XP029475923, ISSN: 0378-5173, DOI: 10.1016/J.IJPHARM.2016.01.076
- MORALES ET AL.: "Surfactants: their critical role in enhancing drug delivery to the lungs", THERAPEUTIC DELIVERY, vol. 2, no. 5, 2011, pages 623 - 641, XP055621156

**Description**

**RELATED APPLICATION**

This application claims the benefit of U.S. Provisional Application No. 62/609,275, filed on December 21, 2017.

**BACKGROUND OF THE INVENTION**

Endogenous pulmonary lung surfactant (LS) reduces surface tension at the air-liquid interface of the alveolar lining, preventing the lungs from collapsing at end expiration. Surfactant deficiency is a common disorder in premature infants and causes respiratory distress syndrome (RDS), which can be effectively treated with preparations which are lipid extracts of minced mammalian lung or lung lavage. Said preparations are known as modified natural surfactants and they are mainly composed of phospholipids (PLs) such as phosphatidylcholine (PC), phosphatidylethanolamine (PE) and phosphatidylglycerol (PG) and the hydrophobic surfactant proteins B and C (SP-B and SP-C). Such preparations are formulated as intratracheal suspensions for administration via an endotracheal tube in the infant patient.

A list of PLs cited in this patent application follows:

- phosphatidylcholine: PC,
- phosphatidylethanolamine: PE,
- phosphatidylglycerol: PG,
- phosphatidylinositol: PI,
- phosphatidylserine: PS,
- sphingomyelin: SM,
- 1,2-dipalmitoyl-sn-glycero-3-phosphoglycerol, generally known as dipalmitoyl-phosphatidylglycerol: DPPG,
- 1,2-dipalmitoyl-sn-glycero-3-phosphocholine, generally known as dipalmitoylphosphatidylcholine: DPPC,
- 1-palmitoyl-2-oleyl-sn-glycero-3-phosphoglycerol, generally known as palmitoyl-oleyl-phosphatidylglycerol: POPG,
- 1-palmitoyl-2-oleyl-sn-glycero-3-phosphocholine generally known as palmitoyl-oleyl-phosphatidylcholine: POPC,
- 1,2-dioleyl-sn-glycero-3-phosphoglycerol generally known as dioleyl-phosphatidylglycerol: DOPG,
- 1-palmitoyl-2-linoleyl-sn-glycero-3-phosphocholine, generally known as palmitoyl-linoleyl-phosphatidylcholine: PLPC,
- 1-stearoyl-2-arachidonoyl-sn-glycero-3-phosphocholine, generally known as stearoyl-arachidonoyl-phosphocholine (SAPC),
- 1-palmitoyl-2-arachidonoyl-sn-glycero-3-phosphocholine (PAPC),
- 1,2-dipalmitoyl-sn-glycero-3-phosphoethanolamine, generally known as dipalmitoyl-phosphatidylethanolamine: DPPE,
- 1,2-distearoyl-sn-glycero-3-phosphoethanolamine, generally known as distearoyl-phosphatidylethanolamine: DSPE,
- 1,2-dipalmitoyl-sn-glycero-3-phospho-L-serine, generally known as dipalmitoyl-phosphatidylserine: DPPS.

The glycerol moieties of the phospholipids are mainly esterified with long chain fatty acids ($C_{14}$-$C_{20}$) which in turn can be saturated (e.g., myristic, palmitic and stearic acid), monounsaturated (e.g., oleic acid) or polyunsaturated (e.g., linoleic and arachidonic acid). Phospholipids containing as the characterizing residues neutral or zwitter-ionic moieties such as glycerol (PG), inositol (PI) and serine (PS) are known as acidic phospholipids. Other examples of acidic phospholipids are DPPG, POPG and DPPS.

Surfactants are usually administered to premature infants in the form of aqueous suspensions by instillation into the lungs through the trachea. They can also be administered to adults affected by various pathologies involving a severe pulmonary insufficiency such as adult respiratory distress syndrome (ARDS).

One of the most important lipid components of surfactant preparations is 1,2-dipalmitoyl-sn-glycero-3-phosphocholine (DPPC) as it forms a monomolecular film at the air-liquid interface during compression, probably undergoing phase transition (solidification) during surface compression, thereby stabilizing a system of alveoli with different sizes. It is also generally recognized that acidic phospholipids are of paramount importance in order to obtain a good activity since they favor the spreading of DPPC.

Surfactant preparations obtained from animal tissues present some drawbacks, like their availability in limited amounts, the complexity of the production and sterilization processes and the relevant production costs: as a consequence, many efforts have been made to prepare synthetic surfactants. "Artificial" surfactants are devoid of surfactant proteins and simply consist of mixtures of synthetic compounds, primarily phospholipids and other lipids that are formulated to mimic the lipid composition and behavior of natural surfactant. "Reconstituted" surfactants are artificial surfactants to which have been added surfactant proteins isolated from animals or manufactured through recombinant technology. Patent document WO 97/26863 discloses a process for the production of powdered pulmonary surfactant preparations.

Another major drawback of most prior art surfactant preparations is that they must be delivered to the patient via an endotracheal tube. It would be desirable to provide a surfactant formulation that is suitable for pulmonary delivery via inhalation, without the need for intubation to an infant or adult with comprised lung function and with enhanced stability properties.

## SUMMARY OF THE INVENTION

The present invention is directed to respirable, dry powder particle formulations of lung surfactants that comprise surfactant proteins and that are formulated for delivery to the pulmonary system via inhalation.

The formulations of the invention comprise active agents selected from the group consisting of one or more surfactant proteins, and may also comprise one or more lung surfactants and/or one or more phospholipids. The claims demand the presence of a surfactant protein selected from the group consisting of SP-A, SP-B, SP-C and SP-D. Preferably the formulations of the invention are free of additional active agents. The formulations are packaged with a desiccant, such as silica gel. Thus, the invention includes a container comprising capsule containing a formulation as described herein packaged in combination with a desiccant.

Claimed is a container comprising: (a) a capsule containing a respirable, dry powder particle surfactant formulation for pulmonary delivery comprising: i) at least 30% DPPC by weight of the particle; ii) 0.1 to 3% NaCl by weight of the particle; iii) 1% to 10% by weight of the particle of a surfactant protein selected from the group consisting of: SP-A, SP-B, SP-C and SP-D, or an active fragment thereof that contains at least a portion of a lipid associating region; and iv) 10% to 30% by weight of an excipient selected from the group consisting of any one or more of leucine, trehalose, lactose, mannitol, sugar alcohol, or a hydrogenated starch hydrolysate (HSH); wherein all components of the dry powder particles amount to 100 weight percent; and (b) a desiccant.

Preferably the formulation further comprises any one or more of DOPC, POPC, DPPE, DPPG POPG or any salt thereof (e.g., DPPG-Na or POPG-Na).

Optionally, the formulation further comprises a fatty acid derived from a vegetable or animal fat. Preferred fatty acids include lauric, myristic, palmitic (PA), stearic, oleic, linoleic and linolenic fatty acids. Preferably the fatty acid is palmitic acid (PA).

The formulation comprises 1% to 10% and preferably 1-5%, such as about 3% or about 5% by weight of a surfactant protein selected from the group consisting of: SP-A, SP-B, SP-C and SP-D; SEQ ID NOs: 1-21 or an active fragment thereof that contains at least a portion of a lipid associating region or any amino acid sequence homologous to any of the foregoing amino acid sequences with at least 90% sequence identity at the amino acid level.

The formulation can further comprise about 1% to about 10% and preferably about 1-5%, such as about 3% or about 5% by weight of sinapultide, also known as "KL4" or other polypeptides, as described in US Patent 5,260,273 by Cochrane et al. issued November 9, 1993. For example, the polypeptide can comprise at least 10 amino acid residues and no more than about 60 amino acid residues, and preferably is in the range of 20-30 residues in length. The polypeptide can include a sequence having alternating hydrophobic and positively charged amino acid residue regions.

Preferably, Leucine is L-leucine.

Weight percent is intended to reflect the total amount of solids, lipids, and/or excipients in the dry particles without regard to residual water, solvent or impurities. All of the components of the dry particles amount to 100 wt %.

## DESCRIPTION OF THE DRAWINGS

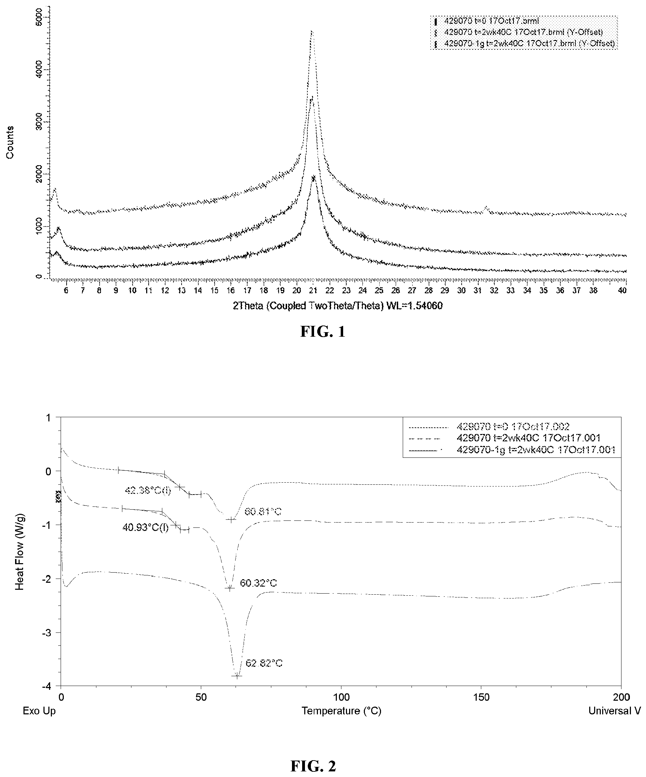

FIG. 1 is an XRPD of Formulation 79 stored with a desiccant over time.
FIG. 2 is a DSC spectrum of Formulation 79 stored with a desiccant over time.

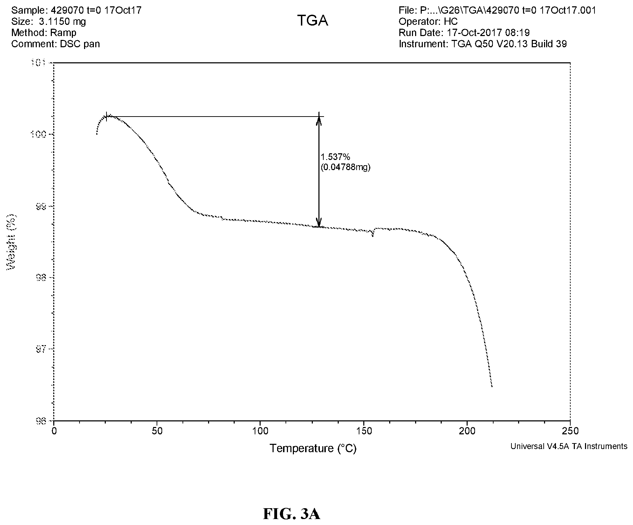

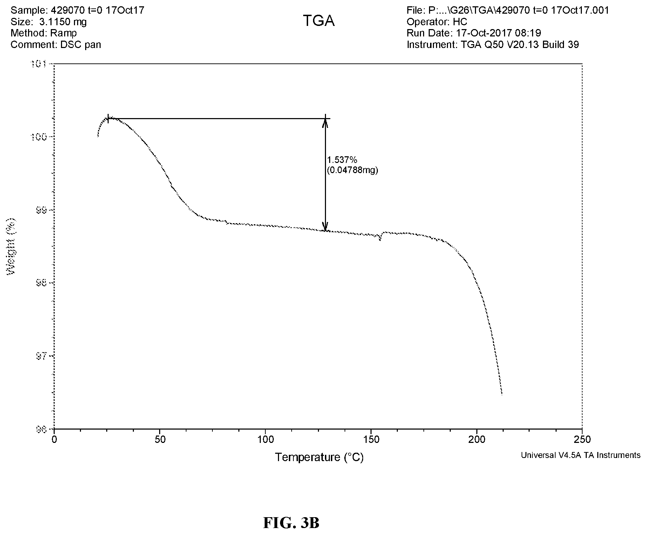

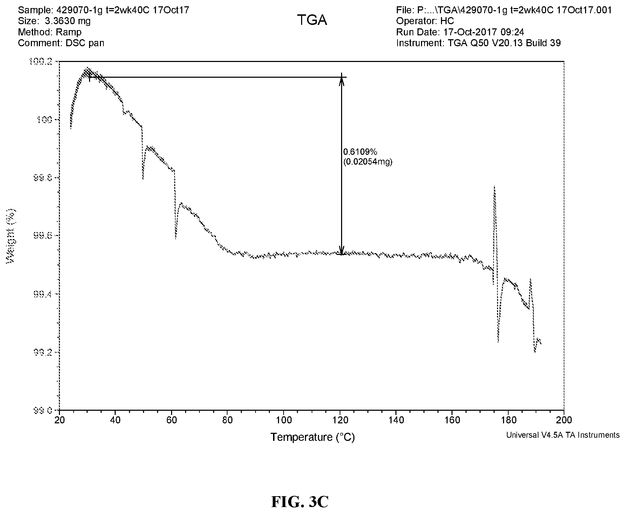

FIG. 3A, 3B and 3C show the TGAs of Formulation 79 over time.

## DETAILED DESCRIPTION OF THE INVENTION

The terms "a", "an" and "the" as used herein are defined to mean "one or more" and include the plural unless the context is inappropriate.

The term "comprising" as used herein which is synonymous with "including," "containing," or "characterized by," is inclusive or open-ended and does not exclude additional, unrecited elements of a composition or method steps. The term "consisting of" excludes any element, step, or ingredient that is not otherwise specified. The term "consisting essentially of" limits the scope of a composition or method to the specified materials or steps and those that do not materially affect the basic and novel characteristic(s) of the specified composition or method.

The term "dry powder" as used herein refers to a composition that contains finely dispersed respirable dry particles that are capable of being dispersed in an inhalation device and subsequently inhaled by a subject. Such dry powder or dry particle may contain up to about 15% water or other solvent, preferably up to about 10% water or other solvent, or preferably be substantially free of water or other solvent, or preferably be anhydrous.

The term "dry particles" as used herein refers to respirable particles that may contain up to about 15% water or other solvent, preferably up to 10% water or other solvent or preferably be substantially free of water or other solvent, or preferably be anhydrous.

The term "respirable" as used herein refers to dry particles or dry powders that are suitable for delivery to the respiratory tract (e.g., pulmonary delivery) in a subject by inhalation. Respirable dry powders or dry particles have a mass median aerodynamic diameter (MMAD) of less than about 10 microns, preferably about 5 microns and more preferably about 3 microns or less. The "mass median aerodynamic diameter" (MMAD) is also referred to herein as "aerodynamic diameter". Experimentally, aerodynamic diameter can be determined by employing a gravitational settling method, whereby the time for an ensemble of powder particles to settle a certain distance is used to infer directly the aerodynamic diameter of the particles. An indirect method for measuring the mass median aerodynamic diameter (MMAD) is the multi-stage liquid impinger (MSLI). The aerodynamic diameter, $d_{aer}$, can be calculated from the equation:

$$d_{aer} = d_g \sqrt{\rho_{tap}}$$

where $d_g$ is the geometric diameter, for example the MMGD, and $\rho$ is the powder density.

As used herein, the terms "administration" or "administering" of respirable dry particles refers to introducing respirable dry particles to the respiratory tract of a subject.

As used herein, the term "respiratory tract" includes the upper respiratory tract (e.g., nasal passages, nasal cavity, throat, pharynx), respiratory airways (e.g., larynx, tranchea, bronchi, bronchioles) and lungs (e.g., respiratory bronchioles, alveolar ducts, alveolar sacs, alveoli). The deep lung, or alveoli, are typically the desired target of inhaled therapeutic formulations for systemic drug delivery. In one embodiment of the invention, most of the mass of particles deposit in the deep lung or alveoli. In another embodiment of the invention, delivery is primarily to the central airways. In other embodiments, delivery is to the upper airways.

"Pulmonary delivery," as that term is used herein refers to delivery to the respiratory tract. Pulmonary delivery includes inhalation by a patient that is capable of independent inhalation or inhalation via a ventilation system such as a mechanical ventilation (MV) system or a non-invasive mechanical ventilation system (NIMV) such as via a continuous positive airway pressure (CPAP) system.

The term "working density" as used herein is interchangeable with the term "bulk density" and is defined herein as the weight of the powder (*m*) divided by the volume it occupies (*Vo*) and is expressed herein as grams per liter (g/L) as determined by measurement in a graduated cylinder. Briefly, a graduated cylinder is first weighed, filled with powder without compacting, leveled if necessary without compacting and weighed again. The unsettled apparent volume is read to the nearest graduated unit. The working density is calculated by the formula *m*/*Vo*. Working density may also be expressed for example in grams per cubic centimeter (g/cm$^3$). In one embodiment, the working density is less than 0.1 g/cm$^3$. In one embodiment, the working density ranges from about 0.02 g/cm$^3$ to about 0.05 g/cm$^3$. In one embodiment, the capsules contain powder with a working density between about 0.03 g/cm$^3$ to about 0.06 g/cm$^3$. In another embodiment, the capsules contain powder with a working density between about 0.04 g/cm$^3$ to about 0.05 g/cm$^3$. In a further embodiment, the capsules contain powder with a working density of about 0.04 g/cm$^3$. In a further embodiment, the capsules contain powder with a working density of about 0.045 g/cm$^3$. In a further embodiment, the capsules contain powder with a working density of about 0.05 g/cm$^3$. In a further embodiment, the capsules contain powder with a working density of about 0.035 g/cm$^3$. In a further embodiment, the capsules contain powder with a working density of about 0.03 g/cm$^3$. In one embodiment, the capsules contain powder with a working density between about 0.03 g/cm$^3$ to about 0.05 g/cm$^3$. In another embodiment, the capsules contain powder with a working density between about 0.04 g/cm$^3$ to about 0.06 g/cm$^3$. In another embodiment, the capsules contain powder with a working density between about 0.05 g/cm$^3$ to about 0.06 g/cm$^3$. In another embodiment, the capsules contain powder with a working density between about 0.06 g/cm$^3$ to about 0.07 g/cm$^3$.

The term "dispersible" is a term of art that describes the characteristic of a dry powder or dry particles to be dispelled into a respirable aerosol. One way of measuring the dispersibility of a dry powder or dry particles is expressed herein as the quotient of the volume median geometric diameter (VMGD) measured at a dispersion (i.e., regulator) pressure of 1 bar divided by the VMGD measured at a dispersion (i.e., regulator) pressure of 4 bar, or VMGD at 0.5 bar divided by the VMGD at 4 bar as measured by HELOS/RODOS. These quotients are referred to herein as "¼ bar," and "0.5/4 bar," respectively, and dispersibility correlates with a low quotient. For example, ¼ bar refers to the VMGD of respirable dry particles or powders emitted from the orifice of a RODOS dry powder disperser (or equivalent technique) at about 1 bar, as measured by a HELOS or other laser diffraction system, divided the VMGD of the same respirable dry particles or powders measured at 4 bar by HELOS/RODOS. Thus, a highly dispersible dry powder or dry particles will have a ¼ bar or 0.5/4 bar ratio that is close to 1.0. Highly dispersible powders have a low tendency to agglomerate, aggregate or clump together and/or, if agglomerated, aggregated or clumped together, are easily dispersed or de-agglomerated as they emit from an inhaler and are breathed in by the subject. Dispersibility can also be assessed by measuring the size emitted from an inhaler as a function of flowrate.

Another way of measuring dispersibility that is of particular relevance to this application is to measure the emitted dose (ED) as a function of decreasing flow rate from a dry powder inhaler (DPI) or related device. Preferably, the LS powders of this invention are capable of being dispersed from a simplified version of a DP device (hereafter referred to as low flow aerosolization chamber, or LFAC, device) consisting of a simple cylindrical chamber with one or more holes at one end that can accommodate a perforated capsule containing powder) at extremely low flow rates (10 liters per minute (lpm) or less) with little resistance provided to aid in dispersion. Powder dispersibility can be quantified via calculating the ratio of the ED of a powder at a relatively low flow rate (i.e., 20, 15, 10 or 5 lpm, etc.) to the ED measured at a standard flow rate of 28.3 lpm. Thus, the LS powders disclosed herein are capable of being dispersed and deagglomerated at greatly decreased energies (a function of device resistance and flow rate through the device) as compared to traditional DP formulations administered via conventional DPI devices.

A high degree of dispersibility is a key advantageous aspect of the LS powders disclosed herein. Traditional dry powder (DP) formulations for pulmonary delivery are administered via a dry powder inhaler (DPI) with a relatively moderate to high degree of resistance incorporated into the device which acts to facilitate the dispersion and deagglomeration of the powder as patients inhale through the device at flow rates ranging from 20 to 60 liters per minute (lpm).

The terms "FPF (<5.6)," "FPF (<5.6 microns)," and "fine particle fraction of less than 5.6 microns" as used herein, refer to the fraction of a sample of dry particles that have an aerodynamic diameter of less than 5.6 microns. For example, a two- or three-stage collapsed ACI can be used to measure FPF < 5.6 microns. The two-stage collapsed ACI consists of only the top stage (S0) and the filter stage of the eight-stage ACI and allows for the collection of two separate powder fractions. Specifically, a two-stage collapsed ACI is calibrated so that the fraction of powder that is collected on S0 is composed of non-respirable dry particles that have an aerodynamic diameter of greater than 5.6 microns. The fraction of powder passing S0 and depositing on the filter stage is thus composed of respirable dry particles having an aerodynamic diameter of less than 5.6 microns. The airflow at such a calibration is approximately 60 L/min. This parameter may also be identified as "FPF TD(<5.6)," where TD means total dose. A similar measurement can be conducted using an eight-stage ACI. The eight-stage ACI cutoffs are different at the standard 60 L/min flowrate, but the FPF TD(<5.6) can be extrapolated from the eight-stage complete data set. The eight-stage ACI result can also be calculated by the USP method of using the dose collected in the ACI instead of what was in the capsule to determine FPF.

The terms "FPF (<3.4)," "FPF (<3.4 microns)," and "fine particle fraction of less than 3.4 microns" as used herein, refer to the fraction of a mass of respirable dry particles that have an aerodynamic diameter of less than 3.4 microns. For example, three-stage collapsed ACI can be used to measure both FPF < 5.6 microns and < 3.4 microns. The three-stage collapsed ACI consists of collection stage S0, S2 and the filter stage and provides fractions of powder of an aerodynamic diameter greater than 5.6 microns, less than 5.6 microns and less than 3.4 microns. This parameter may also be identified as "FPF TD(<3.4)," where TD means total dose. A similar measurement can be conducted using an eight-stage ACI. The eight-stage ACI result can also be calculated by the USP method of using the dose collected in the ACI instead of what was in the capsule to determine FPF. Other cutoff values for FPF (i.e., < 5.0 microns, etc.) can be utilized in a similar manner either via utilizing different stage configurations for the ACI or else extrapolating from the results obtained for a specific set of stages and cutoff diameters.

As used herein, the term "emitted dose" or "ED" refers to an indication of the delivery of a drug formulation from a suitable inhaler device after a firing or dispersion event. More specifically, for dry powder formulations, the ED is a measure of the percentage of powder that is drawn out of a unit dose package and that exits the mouthpiece of an inhaler device. The ED is defined as the ratio of the dose delivered by an inhaler device to the "nominal dose" (i.e., the mass of powder per unit dose placed into a suitable inhaler device prior to firing). The ED is an experimentally-measured parameter, and can be determined using the method of USP Section 601 Aerosols, Metered-Dose Inhalers and Dry Powder Inhalers, Delivered-Dose Uniformity, Sampling the Delivered Dose from Dry Powder Inhalers, United States Pharmacopia Convention, Rockville, Md., 13th Revision, 222-225, 2007. This method utilizes an *in vitro* device set up to mimic patient dosing.

The term "capsule emitted powder mass" or "CEPM" as used herein refers to the amount of dry powder formulation emitted from a capsule or dose unit container during an inhalation maneuver. CEPM is measured gravimetrically, typically by weighing a capsule before and after the inhalation maneuver to determine the mass of powder formulation removed. CEPM can be expressed either as the mass of powder removed, in milligrams, or as a percentage of the initial filled powder mass in the capsule prior to the inhalation maneuver.

The term "effective amount," as used herein, refers to the amount of agent needed to achieve the desired effect, such as an amount that is sufficient to increase surface and/or bulk viscoelasticity of the respiratory tract mucus (e.g., airway lining fluid), increase gelation of the respiratory tract mucus (e.g., at the surface and/or bulk gelation), increase surface tension of the respiratory tract mucus, increasing elasticity of the respiratory tract mucus (e.g., surface elasticity and/or bulk elasticity), increase surface viscosity of the respiratory tract mucus (e.g., surface viscosity and/or bulk viscosity), reduce the amount of exhaled particles, reduce pathogen (e.g., bacteria, virus) burden, reduce symptoms (e.g., fever, coughing, sneezing, nasal discharge, diarrhea and the like), reduce occurrence of infection, reduce viral replication, or improve or prevent deterioration of respiratory function (e.g., improve forced expiratory volume in 1 second FEV1 and/or forced expiratory volume in 1 second FEV1 as a proportion of forced vital capacity FEV1/FVC) and/or reduce bronchoconstriction. The actual effective amount for a particular use can vary according to the particular dry powder or dry particle, the mode of administration, and the age, weight, general health of the subject, and severity of the symptoms or condition being treated. Suitable amounts of dry powders and dry particles to be administered, and dosage schedules, for a particular patient can be determined by a clinician of ordinary skill based on these and other considerations. With respect to an "effective amount" of a desiccant, it is understood that the amount is sufficient to improve the shelf life or stability of a formulation packaged with a desiccant, such as silica gel, typically packaged in a sachet, pouch or pack, as compared to a formulation packaged without.

Preferred "excipients" as that term is used herein are those excipients that can be taken into the lungs with no significant adverse toxicological effects on the lungs. Such excipients are generally regarded as safe (GRAS) by the U.S. Food and Drug Administration. Such excipients include water.

The term "patient" or "subject" as used interchangeably herein are individuals to whom the compositions of the invention may be administered. Examples of such individuals include adult humans and pediatric humans. Pediatric humans include individuals aged from birth up to 18 years of age. Pediatric aged children may also include the following subgroups including but not limited to, neonates comprising newborn individuals up to about 28 days of age or 1 month of age; infants comprising individuals aged from the neonatal period up to 12 months of age; toddlers comprising individuals of ages 1-3 years old; preschool children comprising individuals of ages 3-5 years old, school-aged children comprising individuals of ages 6 to 10 years old and adolescents comprising individuals of ages 11-14 years. Pediatric children may also be referred to having the following age ranges of about 6 to about 11 years of age, about 12 to about 17 years of age, or about 6 to about 17 years of age. Premature human children (preemies) include individuals who are less than about 37 weeks of gestational age.

The term "compromised patients" as used herein includes individuals who do not or cannot breathe hard or have a compromised lung function. Examples of such individuals include premature infants and/or newborns suffering from respiratory distress syndrome (RDS), adults suffering from acute respiratory distress (ARDS), and adults and children suffering from other disease states associated with compromised lung function (cystic fibrosis, COPD, etc.). Generally, the individual will have a peak inspiratory flow rate (PIFR) of less than about 30 liters per minute. In one embodiment, the patient will have a PIFR of about 15 liters per minute or less. Alternatively or additionally, the compromised patient has an inspiration volume of less than 2 liters, such as less than about 1.5 liters, including less than about 1 liter, such as about 0.75 liters, such as about 0.5 liters, such as about 0.2 liters, or such as about 0.1 liters or less.

The term "peak inspiratory flow rate" (PFIR) as used herein refers to a patient's maximum speed of inhalation as conventionally assessed via a pulmonary flow meter.

The term "hydrogenated starch hydrolysate" (HSH) or "polyglycitol" as used interchangeably herein refers to the broad group of polyols that contain substantial quantities of hydrogenated oligo- and polysaccharides in addition to any monomeric or dimeric polyols (sorbitol, mannitol or maltitol, respectively). HSH are produced by the partial hydrolysis of corn, wheat or potato starch and subsequent hydrogenation of the hydrolysate at high temperature under pressure. The end product is an ingredient composed of sorbitol, maltitol and higher hydrogenated saccharides (maltitritol and others). By varying the conditions and extent of hydrolysis, the relative occurrence of various mono-, di-, oligo- and polymeric hydrogenated saccharides in the resulting product can be obtained.

Sequences similar to or homologous to (e.g., at least about 70% sequence identity) the sequences disclosed herein are also part of the invention. In some embodiments, the sequence identity at the amino acid level can be about 70%, 80%, 85%, 90%, 91%, 92%, 93%, 94%, 95%, 96%, 97%, 98%, 99% or higher. At the nucleic acid level, the sequence identity can be about 70%, 75%, 80%, 85%, 90%, 91%, 92%, 93%, 94%, 95%, 96%, 97%, 98%, 99% or higher. Alternatively, substantial identity exists when the nucleic acid segments will hybridize under selective hybridization conditions (e.g., very high stringency hybridization conditions), to the complement of the strand. The nucleic acids may be present in whole cells, in a cell lysate, or in a partially purified or substantially pure form.

Calculations of "homology" or "sequence identity" or "similarity" between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "homology" is equivalent to amino acid or nucleic acid "identity"). The percent identity between the two sequences is a function of the number of identical positions shared by the sequences, taking into account the number of gaps, and the length of each gap, which need to be introduced for optimal alignment of the two sequences. In the case of circularly related proteins, the sequence of one of the partners needs to be appropriately split and aligned in two sections to achieve optimal alignment of the functionally equivalent residues necessary to calculate the percent identity.

Amino acid and nucleotide sequence alignments and homology, similarity or identity, as defined herein are preferably prepared and determined using the algorithm BLAST 2 Sequences, using default parameters (Tatusova, T. A. et al., FEMS Microbiol Lett, 174:187-188 (1999)). Alternatively, the BLAST algorithm (version 2.0) is employed for sequence alignment, with parameters set to default values. BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul, 1990, Proc. Natl. Acad. Sci. USA 87(6):2264-8.

There are several important limitations related to the potential implementation of LS delivery therapies for the treatment of neonatal RDS in the developing world are related to the costs, resources, equipment and infrastructure required to deliver replacement LS in liquid form via intratracheal instillation to premature infants as such formulations cannot be delivered effectively via passive nebulization. A dry powder formulation of LS could instead be delivered in a noninvasive manner, such as via incorporation of a dry powder LS formulation and delivery system into a noninvasive ventilation system such as a continuous positive airway pressure (CPAP) system. Such a formulation and delivery system could thus provide an inexpensive and efficacious alternative for use in developing areas where intratracheal liquid installation is not possible and where LS formulations must be in a more stable and portable dry powder form versus in a liquid form that requires refrigeration.

The invention provides respirable dry powder particles that comprise one or more lung surfactants (LS), one or more phospholipids, and optionally one or more lung surfactant proteins as active ingredients. Preferably the formulations are free of additional active agents.

The invention further provides for packaging respirable dry powders in the presence of a desiccant, such as silica gel desiccant; a zeolite; an alumina; a bauxite; anhydrous calcium sulphate; water-absorbing clay; a molecular sieve; and any mixtures thereof. The desiccant can be packaged in a sachet, pouch or pack and placed in the packaging with the capsules. The desiccant can also be integrated into the packaging itself, such as by coating, absorption or adsorption. For example, a film sealing a blister pack containing a capsule can comprise the desiccant, such as by integration, absorption or adsorption. The packaging can be further sealed with a moisture barrier such as a bottle or blister with a foil seal.

The desiccant will be added in an amount effective to reduce moisture within the packaging during storage and will depend on the size of the container and the internal exposure conditions to humidity. For example, 1g of silica gel is sufficient to protect a package containing 5 Size 00 capsules (HPMC capsules). It is preferred that the humidity, or volatiles content, within the packaging is reduced to less than 5wt%, preferably less than 3 wt%, such as less than 1 wt%. In a preferred embodiment, the amount of desiccant is added to maintain the FPF value after 2 weeks, such as 4 weeks, of storage at 40°C. The term "maintains the FPF value" is intended to mean that the FPF <5.6 microns after storage is at least about 50%, preferably >55%, >60%, >65%, >70%, >75%, or >80%, of the FPF <5.6 microns of a control capsule tested from the same production lot before storage.

The respirable dry powder particles of the invention are particularly formulated for inhalation by patients having compromised lung function also referred to herein as "compromised patients" such as those patients suffering from surfactant deficiency resulting from a disease condition. The dry powder particles of the invention are capable of being inhaled through, for example a dry powder inhaler, or via a system that provides inhalation through a ventilator.

The respirable, dry powder particle surfactant formulation for pulmonary delivery comprises:
i) at least 30% DPPC by weight of the particle; ii) 0.1 to 3% NaCl by weight of the particle; iii) 1% to 10% by weight of the particle of a surfactant protein selected from the group consisting of: SP-A, SP-B, SP-C and SP-D, or an active fragment thereof that contains at least a portion of a lipid associating region; and iv) 10% to 30% by weight of an excipient selected from the group consisting of any one or more of leucine, trehalose, lactose, mannitol, sugar alcohol, or a hydrogenated starch hydrolysate (HSH); wherein all components of the dry powder particles amount to 100 weight percent.

Preferably the formulation further comprises one or more of additional phospholipids, such as DOPC, POPC, DPPE, DPPG POPG or any salt thereof (e.g. DPPG-Na or POPG-Na). Preferably, the formulation comprises a combination of DPPC, POPG, and/or POPC or salts thereof. In one embodiment the combination is DPPC and POPG or a salt thereof. POPG-Na is a preferred additional phospholipid.

The total phospholipids (DPPC and additional phospholipid, if any) is at least 30% by weight, preferably at least about 40% by weight, more preferably at least 50% by weight, preferably at least about 60% by weight, more preferably at least 70% by weight, such as about 80% by weight. The ratio of DPPC to additional phospholipids is preferably at least 1:1. For example, the ratio of DPPC: additional phospholipids (e.g., POPG or POPG-Na) can be between about 1:1 to 4:1 (e.g.,1:1, 2:1, 3:1, 4:1). Preferably, the ratio is about 4:1 or less. For example, the ratio of DPPC: POPG-Na can be more than about 7:3, such as between about 7:3 to 3:1.

Optionally, the formulation further comprises a fatty acid derived from a vegetable or animal fat. Preferred fatty acids include lauric, myristic, palmitic (PA), stearic, oleic, linoleic and linolenic fatty acids. Preferably the fatty acid is palmitic acid (PA).

The formulation comprises 1% to 10% and preferably 1% to about 5%, such as about 3% or 5%, by weight of a surfactant protein selected from the group consisting of: SP-A, SP-B, SP-C and SP-D; SEQ ID NOs: 1-21, or an active fragment thereof that contains at least a portion of a lipid associating region or any amino acid sequence homologous to any of the foregoing amino acid sequences with at least 70%, 80%, 85%, 90% or 95% sequence identity at the amino acid level.

The formulation can further comprise about 1% to about 10% and preferably about 1-5%, such as about 3% or about 5% by weight of sinapultide, also known as "KL4" or other polypeptides, as described in US Patent 5,260,273 by Cochrane et al., issued November 9, 1993. For example, the polypeptide can comprise at least 10 amino acid residues and no more than about 60 amino acid residues, and preferably is in the range of 20-30 residues in length. The polypeptide can include a sequence having alternating hydrophobic and positively charged amino acid residue regions.

Preferably, Leucine is L-leucine.

Weight percent is intended to reflect the total amount of solids, lipids, and/or excipients in the dry particles without regard to residual water, solvent or impurities. Preferably, all of the components of the dry particles amount to 100 wt %.

The respirable dry particles of the invention preferably have an MMAD of about 10 microns or less, such as an MMAD of about 0.5 micron to about 10 microns. Preferably, the dry particles of the invention have an MMAD of about 7 microns or less (e.g., about 0.5 micron to about 7 microns), preferably about 1 micron to about 7 microns, or about 2 microns to about 7 microns, or about 3 microns to about 7 microns, or about 4 microns to about 7 microns, about 5 microns to about 7 microns, about 1 micron to about 6 microns, about 1 micron to about 5 microns, about 2 microns to about 5 microns, about 2 microns to about 4 microns, or about 3 microns.

The fine particle fraction less than 5.6 microns, or FPF<5.6 of a powder corresponds to the percentage of particles in the powder that have an aerodynamic diameter of less than 5.6 $\mu$m. The FPF<5.6 of a powder of the invention is preferably about 40% or more. In certain embodiments, the FPF<5.6 of the powder is at least about 50%, 60% or 70%. In one embodiment, the FPF<5.6 is about 30% to about 90%. In one embodiment, the FPF<5.6 is about 70% to about 95%. In one embodiment, the FPF<5.6 is about 70% to about 90%. In one embodiment, the FPF<5.6 is about 70% to about 85% or about 70% to about 80%.

The fine particle fraction less than 3.4 microns, or FPF<3.4, of a powder corresponds to the percentage of particles in the powder that have an aerodynamic diameter of less than 3.4 $\mu$m. In one embodiment, the FPF<3.4 of a powder of the invention is about 30% or more. In one embodiment, the FPF<3.4 of the powder is at least about 40% or 50%. In one embodiment, the FPF<3.4 is about 30% to 60%.

Preferably, the powders of the invention have a tap density of less than about 0.4 g/cm$^3$. For example, the powders have a tap density between 0.02 and 0.20 g/cm$^3$, between 0.02 and 0.15 g/cm$^3$, between 0.03 and 0.12 g/cm$^3$, between 0.05 and 0.15 g/cm$^3$, or less than about 0.15 g/cm$^3$, or a tap density less than about 0.10 g/cm$^3$, a tap density less than about 0.15 g/cm$^3$. In one embodiment, the powders of the invention have a tap density of less than about 0.2 g/cm$^3$. Preferably, the tap density is from about 0.02 to 0.175 g/cm$^3$. Preferably, the tap density is from about 0.06 to 0.175 g/cm$^3$.

Tap density can be measured by using instruments known to those skilled in the art such as the Dual Platform Microprocessor Controlled Tap Density Tester (Vankel, N.C.) or a GEOPYC $^{TM}$ instrument (Micrometrics Instrument Corp., Norcross, GA, 30093). Tap density is a standard measure of the envelope mass density. Tap density can be determined using the method of USP Bulk Density and Tapped Density, United States Pharmacopia convention, Rockville, Md., 10th Supplement, 4950-4951, 1999. Features which can contribute to low tap density include irregular surface texture and porous structure. The envelope mass density of an isotropic particle is defined as the mass of the particle divided by the minimum sphere envelope volume within which it can be enclosed. In one embodiment of the invention, the particles have an envelope mass density of less than about 0.4 g/cm$^3$.

Preferably, the respirable dry powders and dry particles formulations of the invention have a water or solvent content of less than about 15% by weight, less than about 13% by weight, less than about 11.5% by weight, less than about 10% by weight, less than about 9% by weight, less than about 8% by weight, less than about 7% by weight, less than about 6% by weight, less than about 5% by weight, less than about 4% by weight, less than about 3% by weight, less than about 2% by weight, less than about 1% by weight or be anhydrous.

Preferably, the dry particle formulations of the invention can have a water or solvent content of less than about 6% and greater than about 1%, less than about 5.5% and greater than about 1.5%, less than about 5% and greater than about 2%, about 2%, about 2.5%, about 3%, about 3.5%, about 4%, about 4.5%, or about 5%.

Optional excipients can include sugars, starches, polysaccharides or carbohydrates. For example, sugar alcohols, such as mannitol, sorbitol, maltitol can be used. Additionally or alternatively, lactose, maltose, sucrose, trehalose, raffinose and the like can be used. Carbohydrates such as maltodextrin and cyclodextrins can be used. Preferably, the excipient is added in an amount less than about 40% by weight, preferably less than about 30% by weight, preferably less than about 25% by weight in a formulation. Preferred ranges of excipient include, but are not limited to, about 5 to about 40% by weight, about 10 to about 30% by weight and about 15 to about 25% by weight. More preferably the excipient is added in an amount of about 15% or 18% by weight.

Preferably, the HSH powder can be, for example the polyglycitol, STABILITE™ SD30 or SD60 (INNOVA, Muscatine, Iowa). The polyol distribution for STABILITE™ SD30 is about 2% sorbitol by weight and about 6% maltitol by weight and the polyol distribution for STABILITE™ SD60 is about 1% sorbitol and about 3.5% maltitol. Other general characteristics of the STABILITE™ family of products are listed in Table A.

See, https://www.scribd.com/document/239956170/STABILITE-SD-polyglycitol-powder-fact-sheet.

Table A

| General Characteristics of the STABILITE ™ Products | | |
|---|---|---|
| | STABILITE SD30 | STABILITE SD60 |
| Moisture | 8% maximum | 8% maximum |
| Ash | 0.1% maximum | 0.1% maximum |
| pH of 20% solution | 4.0-5.0 | 4.0-5.0 |
| Reducing Sugars | 1.0% maximum | 1.0% maximum |
| Viscosity of 50% solution at 25° C. | about 90 cP | about 280 cP |
| Osmolality of 20% solution | About 278 mOsm | About 185 mOsm |
| Polyol distribution | HP 1 (sorbitol) ~2% | HP 1 (sorbitol) ~1% |
| | HP 2 (maltitol) ~6% | HP 2 (maltitol) ~3.5% |
| | HP 3 and above ~92% | HP 3 and above ~95.5% |

Preferably, HSH powder is a mixed polyol composition where no one polyol is present in a quantity greater than 50% by weight. Preferably the HSH (preferably, SD30) is less than about 40% by weight, preferably less than about 30% by weight, preferably less than about 25% by weight in a formulation. Preferred ranges of HSH (e.g., SD30) include, but are not limited to, about 5 to about 40% HSH (e.g., SD30) by weight, about 10 to about 30% HSH by weight and about 15 to about 25% by weight.

Any salt is suitable for use in the invention. Preferably less than about 10% by weight, preferably less than about 5% by weight, preferably less than about 3% by weight, preferably less than about 1% by weight, preferably less than about 1 % by weight and preferably between about 0.1% to about 2 % by weight of a salt is present in a formulation of the invention. Preferred ranges of salts include, but are not limited to, about 0.1 to about 10% salt by weight, about 0.1 to about 3% salt by weight and about 0.1 to about 3% by weight. Preferably the particles of the invention comprise about 2% by weight of salts. Preferred salts include, but are not limited to sodium salts, potassium salts, lithium salts and calcium salts. A preferred salt is sodium chloride (NaCl). Preferably the dry particles comprise about 2% by weight NaCl. The claims demand the presence of 0.1 to 3% NaCl by weight of the particle.

The particles of the invention can optionally comprise a fatty acid in an amount ranging from about 1% by weight to about 10% by weight, more preferably from about 1% by weight to about 5% by weight, and more preferably about 1 % by weight, 2% by weight, 3% by weight, 4% by weight or 5% by weight.

Surfactant proteins (SP) are included in the dry powder surfactant formulations of the invention. The surfactant proteins include SP-A, SP-B, SP-C, SP-D, SEQ ID NOS: 1-21 or an active fragment thereof that contains at least a portion of a lipid associating region. SP-A and SP-B are believed to have roles in the production of the surfactant monolayer in the lungs. SP-A and SP-D are also believed to have rules are believed to have roles in binding pathogens and other organic materials such as pollens and dust mite antigens that may be present in the lungs.

The structural features of the full-length mature SP-A, SP-B, and SP-C proteins are well known and reported as Genbank Accession Nos. L10123, BC111570, BC111571, BC026229, NM-006926, and NM-005411 for SP-A; L11573, AF400074, BC032785, NM-000542, and NM 198843 for SP-B; and J03890, U02948, AY357924, AY337315, BC005913, and NM-003018 for SP-C. When fragments of the mature SP-A, SP-B, and/or SP-C are employed in the surfactant compositions of the present invention, it is preferable to utilize fragments thereof that contain at least a portion of a lipid associating region. Lipid associating regions are those portions of the mature protein that are capable of molecular interaction with lipids (either native glycerophospholipids or synthetic phospholipase-resistant lipids) to promote surface activity of the resulting composition in which they are introduced. Such fragments include, without limitation, fragments of SP-A that contain an amphipathic or hydrophobic region capable of associating with lipids, fragments of SP-B that contain an amphipathic or hydrophobic region capable of associating with lipids, fragments of SP-C that contain an amphipathic or hydrophobic region capable of associating with lipids, as well as any number of synthetic peptides or combinations thereof. One exemplary SP-B peptide family is designated the "Mini-B Family" (Protein Data Bank Coordinate accession number ISSZ). Exemplary peptides from the Mini-B Family include, but are not limited to SEQ ID Nos: 1-21.

Examples of preferable peptide derivatives of SP-B and SP-C include, but are not limited to those described in U.S. Pat. No. 8,563,683,

including but not limited to the following exemplary peptides of Table B:

Table B

| |
|---|
| CWFCRFFFKRFFFFFPKGGRFFPFFFCRFFFRCS (SEQ ID NO: 1), |
| CWFCRAFIKRFQAMIPKGGRMLPQLVCRLVLRCS (SEQ ID NO: 2), |
| CWLCRALIKRIQAMIPKGGRMFPQFFCRFFFRCS (SEQ ID NO: 3), |
| CWFCRAFIKRFQAMIPKGGRMFPQFFCRFFFRCS (SEQ ID NO: 4), |
| CWFCRAFIKRFQAMIPKGERMLPQLVCRLVLRCS (SEQ ID NO: 5), |
| CWLCRALIKRIQAMIPKGERMFPQFFCRFFFRCS (SEQ ID NO: 6), |
| CWFCRAFIKRFQAMIPKGERMFPQFFCRFFFRCS (SEQ ID NO: 7), |
| CWLCRALIKRIQAMIPCGGRMLPQL VCRL VLRCS (SEQ ID NO: 8), |
| FPIPLPYCWLCRALIKRIQAMIPKGGRMLPQLVCRLVLRCS (SEQ ID NO: 9), |
| FPCPLPYCWLCRALIKRIQAMIPKGGRMLPQLVCRLVLRCS (SEQ ID NO: 10), |
| CPIPLPYCWLCRALIKRIQAMIPKGGRMLPQLVCRLVLRCS (SEQ ID NO: 11), |
| CWLCRALIKRIQAMIPKGALAVAVAQVCRVVPLVAGGICQALAERYS VILLDTLLGRMLPQLVCRLVLRCS (SEQ ID NO: 12). |
| CWLCRALIKRIQAMIPKGALAVAVAQVCRVVPLVAGGICQFLAERYSV ILLDTLLGRMLPQLVCRLVLRCS (SEQ ID NO: 13), |
| FPIPLPYCWLCRALIKRIQAMIPKGALAVAVAQVCRVVPLVVGGICQYL AERYSVILLDTLLGRMLPQLVCRLVLRCS (SEQ ID NO: 14), |
| CPIPLPYCWLCRALIKRIQAMIPKGALAVAVAQVCRVVPLVVGGICQY LAERYSVILLDTLLGRMLPQLVCRLVLRCS (SEQ ID NO: 15), |
| FPCPLPYCVVLCRALIKRIQAMIPKGALAVAVAVCRVVPLVVGGICQY LAERYSVILLDTLLGRMLPQLVCRLVLRCS (SEQ ID NO: 16), |
| FGIPFFPVHLKRLLVVVVVVVLVVVVIVGALLMGL (SEQ ID NO: 17), |
| FGIPFFPVHLKRLLVPVVVVVLVVVVIVGALLMGL (SEQ ID NO: 18), |
| FGIPFFPVHLKRLLVVVVVPVLVVVVIVGALLMGL (SEQ ID NO: 19), |
| FGIPFFPVHLKRLLVVVVVVVLVPVVIVGALLMGL (SEQ ID NO: 20), |
| FGIPFFPVHLKRLLVVVVVVVLVVVVIPGALLMGL (SEQ ID NO: 21), |

and any amino acid sequences homologous to any of the foregoing peptides with at least 70%, preferably at least 80%, preferably at least 85%, preferably at least 90%, preferably at least 95% sequence identity at the amino acid level.

A preferred SP-B peptide for use in the surfactant compositions of the invention comprises the amino acid sequence of SEQ ID NO: 9 or any amino acid sequences homologous thereto with at least 70%, preferably at least 80%, preferably at least 90%, preferably at least 95% sequence identity at the amino acid level.

The formulation can further comprise about 1% to about 10% and preferably about 1-5%, such as about 3% or about 5% by weight of sinapultide, also known as "KL4" or other polypeptides, as described in US Patent 5,260,273 by Cochrane et al. issued November 9, 1993. For example, the polypeptide can comprise at least 10 amino acid residues and no more than about 60 amino acid residues, and preferably is in the range of 20-30 residues in length. The polypeptide can include a sequence having alternating hydrophobic and positively charged amino acid residue regions. The polypeptide can be represented by the formula $(Z_a U_b)_c Z_d$, wherein:

Z is a positively charged amino acid residue, preferably one R or K;

U is a hydrophobic amino acid residue independently selected from the group consisting of V, I, L, C, Y and F. A preferred hydrophobic residue is L;

The average value of a is about 1 to about 5, and is preferably 1. The average value of b is about 3 to about 20, and is preferably in the range of 4-8, and more preferably is about 4. The value of c is 1 to 10, and is preferably in the range of 4-8, and more preferably is about 4. The value of d is 0 to 3, and is preferably 1.

The dry powder particle formulations of the invention can be administered with low inhalation energy such as to compromised patients. In order to relate the dispersion of powder at different inhalation flow rates, volumes, and from inhalers of different resistances, the energy required to perform the inhalation maneuver can be calculated Inhalation energy can be calculated from the equation $E=R^2Q^2V$ where E is the inhalation energy in Joules, R is the inhaler resistance in $kPa^{1/2}/LPM$, Q is the steady flow rate in L/min and V is the inhaled air volume in L.

The dry powder particle formulations of the invention are characterized by a high emitted dose (e.g., CEPM of at least 75%, at least 80%, at least 85%, at least 90%, at least 95%, preferably at least 100%) from a dry powder inhaler when a total inhalation energy of less than about 2 Joules or less than about 1 Joule, or less than about 0.8 Joule, or less than about 0.5 Joule, or less than about 0.3 Joule is applied to the dry powder inhaler. For example, an emitted dose of at least 75%, at least 80%, at least 85%, at least 90%, at least 95% CEPM of a formulation of the invention contained in a unit dose container, containing about 5 to about 50 mg, preferably about 10 mg to about 40 mg, preferably about 25 mg to about 50 mg, preferably about 40 mg, or preferably about 50 mg of the appropriate formulation, in a dry powder inhaler can be achieved when a total inhalation energy of less than about 1 Joule (e.g., less than about 0.8 Joule, less than about 0.5 Joule, less than about 0.3 Joule) is applied to the dry powder inhaler. An emitted dose of at least about 70% CEPM of respirable dry powder contained in a unit dose container, containing about 50 mg or about 40 mg of the respirable dry powder, in a dry powder inhaler can be achieved when a total inhalation energy of less than about 0.28 Joule is applied to the dry powder inhaler. The dry powder can fill the unit dose container, or the unit dose container can be at least 40% full, at least 50% full, at least 60% full, at least 70% full, at least 80% full, or at least 90% full. The unit dose container can be a capsule (e.g., size 000, 00, 0E, 0, 1, 2, 3, and 4, with respective volumetric capacities of 1.37 ml, 950 µl, 770 µl, 680 µl, 480 µl, 360 µl, 270 µl, and 200 µl). Alternatively, the unit dose container can be a blister. The blister can be packaged as a single blister, or as part of a set of blisters, for example, 7 blisters, 14 blisters, 28 blisters, or 30 blisters.

Alternatively, the dry powder formulations of the invention may be administered to patients indirectly via a ventilator. For example, premature infants are often on some kind of ventilation such as invasive mechanical ventilation, or non-invasive ventilation which does not require the use of an endotracheal tube. In such cases, inhalable drugs such as the dry powder formulations of the invention may be administered via a system wherein an inhaler or other device configured to release the dry powder of the invention is incorporated into the ventilation system and is activated to deliver the inhalable drug to the respiratory system of the patient in conjunction with the action of the ventilation system. The formulations of the invention are particularly useful in conjunction with non-invasive ventilation, such as a continuous positive airway pressure (CPAP) system.

An advantage of the invention is the production of powders that disperse well across a wide range of flowrates and are relatively flowrate independent. The dry particles and powders of the invention enable the use of a simple, passive DPI for a wide patient population or alternatively, for use with ventilators of all types and particularly non-invasive ventilation.

The methods (not claimed) comprise administering an effective amount of a surfactant dry powder formulation of the invention to a patient who is need of restoration of pulmonary function as the result of disease characterized by insufficient lung surfactant production. Preferably, the method is effective for treatment of newborns, infants, children, and adults who suffer from any condition caused by insufficient surfactant production.

Abnormalities of surfactant production have been described in various lung diseases including but not limited to asthma, bronchitis, chronic obstructive pulmonary disease, and following lung transplantation. Abnormal surfactant production has also been seen in infectious and suppurative lung diseases, such as cystic fibrosis, pneumonia, and AIDS. Finally, insufficient surfactant also characterizes diseases such as acute respiratory distress syndrome (ARDS), pulmonary edema, interstitial lung diseases, pulmonary alveolar proteinosis, following cardiopulmonary bypass, and in smokers.

The respirable dry particles and dry powder formulations of the invention can be administered to the respiratory tract of a subject in need thereof using any suitable method, such as instillation techniques, and/or an inhalation device, such as a dry powder inhaler (DPI) or metered dose inhaler (MDI). A number of DPIs are available, such as, the ARCUS inhalers (e.g., the inhalers described in US Patent No. 94687288), the inhalers disclosed in U.S. Pat. Nos. 4,995,385 and 4,069,819, SPINHALER® (Fisons, Loughborough, U.K.), ROTAHALERS®, DISKHALER® and DISKUS® (GlaxoSmithKline, Research Triangle Technology Park, N.C.), FLOWCAPSS® (Hovione, Loures, Portugal), INHALATORS® (Boehringer-Ingelheim, Germany), AEROLIZER® (Novartis, Switzerland), and others known to those skilled in the art.

Generally, inhalation devices (e.g., DPIs) are able to deliver a maximum amount of dry powder or dry particles in a single inhalation, which is related to the capacity of the blisters, capsules (e.g. size 000, 00, 0E, 0, 1, 2, 3, and 4, with respective volumetric capacities of 1.37 ml, 950 μl, 770 μl, 680 μl, 480 μl, 360 μl, 270 μl, and 200 μl) or other means that contain the dry particles or dry powders within the inhaler. Accordingly, delivery of a desired dose or effective amount may require two or more inhalations. Preferably, each dose that is administered to a subject in need thereof contains an effective amount of respirable dry particles or dry powder and is administered using no more than about 4 inhalations. For example, each dose of respirable dry particles or dry powder can be administered in a single inhalation or 2, 3, or 4 inhalations. The respirable dry particles and dry powders, are preferably administered in a single, breath-activated step using a breath-activated DPI. When this type of device is used, the energy of the subject's inhalation both disperses the respirable dry particles and draws them into the respiratory tract.

The respirable dry particles or dry powders can be delivered by inhalation to a desired area within the respiratory tract, as desired. It is well-known that particles with an aerodynamic diameter of about 1 micron to about 3 microns, can be delivered to the deep lung. Larger aerodynamic diameters, for example, from about 3 microns to about 5 microns can be delivered to the central and upper airways.

Any one of the above inhalers may also be used in conjunction with an invasive mechanical ventilation (MV) or noninvasive mechanical ventilation (NIMV) such as that which is generally used for premature babies and other individuals suffering from compromised lung function. A noninvasive mechanical ventilation (NIMV) system includes a CPAP system. There is currently no commercially available system designed specifically for inhalation therapy during NIMV. However, the formulations of the present invention are capable of high emitted doses in conjunction with a ventilation system and therefore studies have shown that various inhalers may be configured for use with and to achieve delivery of therapeutically effective amounts to the lung of the patient.

Preferably, a therapeutically effective dosage comprises about 10 mg surfactant formulation per kg of body weight to about 200 mg surfactant formulation per kg of body weight.

Preferred surfactant formulations are shown in Table C Only formulations #72,75,77,82-84,107,109-112 are according to the claims.

Table C

| Formulation # | Description |
|---|---|
| 1 | DPPC:DOPC:POPC:SD-30:NaCl (50:10:10:28:2) |
| 2 | DPPC:DOPC:POPC:DPPE:DPPG-Na:POPG-Na:SD-30:NaCl (32.8:13.3:13.3:2.6:4.0:4.0:28:2.0) |
| 3 | DPPC:DPPG-Na:POPG-Na:CaCl2:SD-30:NaCl (50:10:10:6.9:21.1:2) |
| 4 | DPPC:POPC:POPG-Na:SD-30:NaCl (42:14:14:28:2) |
| 5 | DPPC:POPC:POPG-Na:CaCl2:SD-30:NaCl (42:14:14:5:23:2) |
| 6 | DPPC:POPC:POPG-Na:Mg Lactate:NaCl (42:14:14:28:2) |
| 7 | DPPC:POPC:POPG-Na:SD-30:NaCl (40:24:16:18:2) |
| 8 | DPPC:POPC:POPG-Na:Leu:NaCl (40:24:16:18:2) |
| 9 | DPPC:POPC:POPG-Na:SD-30:NaCl (44:18:18:18:2) |
| 10 | DPPC:POPC:DPPG-Na:POPG-Na:SD-30:NaCl (44:18:9:9:18:2) |
| 11 | DPPC:POPC:POPG-Na:Leu:NaCl (44:18:18:18:2) |
| 12 | DPPC:POPC:DPPG-Na:POPG-Na:Leu:NaCl (44:18:9:9:18:2) |
| 13 | DPPC:POPG-Na:SD-30:NaCl (56:24:18:2) |
| 14 | DPPC:DPPG-Na:POPG-Na:SD-30:NaCl (56:8:16:18:2) |
| 15 | DPPC:POPG-Na:Leu:NaCl (56:24:18:2) |
| 16 | DPPC:DPPG-Na:POPG-Na:Leu:NaCl (56:8:16:18:2) |
| 17 | DPPC:DPPE:DPPG-Na:POPG-Na:SD-30:NaCl (40:24:8:8:18:2) |
| 18 | DPPC:DPPE:DPPG-Na:POPG-Na:Leu:NaCl (40:24:8:8:18:2) |
| 19 | DPPC:POPC:Leucine:NaCl (60:20:18:2) |
| 20 | DPPC:Trehalose:NaCl (80:18:2) |

(continued)

| Formulation # | Description |
|---|---|
| 21 | DPPC:DPPG:POPG:Albumin*:NaCl (58:10:25:5:2) |
| 22 | DPPC:POPG:Albumin* (80:15:5) |
| 23 | DPPC:POPG:Albumin* (70:25:5) |
| 24 | DPPC:POPG:Albumin* (60:35:5) |
| 25 | DPPC:POPG:Albumin*:NaCl (78:15:5:2) |
| 26 | DPPC:POPG:Albumin*:NaCl (68:25:5:2) |
| 27 | DPPC:POPG:Albumin*:NaCl (58:35:5:2) |
| 28 | DPPC:SD-30:Leu:NaCl (40:40:18:2) |
| 29 | DPPC:SD-30:NaCl (80:18:2) |
| 30 | DPPC:SD-30:NaCl (60:38:2) |
| 31 | DPPC: L–leu:NaCl (80:18:2) |
| 32 | DPPC: L–leu:NaCl (60:38:2) |
| 33 | DPPC:DOPC:Leu:NaCl (70:10:18:2) |
| 34 | DPPC:DOPC:SD-30:Leu:NaCl (60:10:20:8:2) |
| 35 | DPPC:DOPC:SD-30:Leu:NaCl (60:20:10:8:2) |
| 36 | DPPC:DOPC:SD-30:NaCl (80:10:8:2) |
| 37 | DPPC:Leu:NaCl (80:18:2) |
| 40 | DPPC:Albumin*:NaCl (93:5:2) |
| 41 | DPPC:Lactose:NaCl (80:18:2) |
| 42 | DPPC:Mannitol:NaCl (80:18:2) |
| 43 | DPPC:POPC:NaCl (70:28:2) |
| 45 | DPPC:POPG:Albumin* (50:45:5) |
| 65 | DPPC:POPG-Na:SD-30:NaCl (49:21:28:2) |
| 66 | DPPC:POPG-Na:Leu:NaCl (49:21:28:2) |
| 67 | DPPC:POPC:SD-30:NaCl (49:21:28:2) |
| 68 | DPPC:POPC:Leu:NaCl (49:21:28:2) |
| 69 | DPPC:POPC:SD-30:NaCl (56:24:18:2) |
| 70 | DPPC:POPC:Leu:NaCl (56:24:18:2) |
| 71 | DPPC:POPG-Na:SD-30:Albumin*:NaCl (49:21:25:3:2) |
| 72 | DPPC:POPG-Na:SD-30:SP-B:NaCl (49:21:25:3:2) |
| 73 | DPPC:POPG-Na:Palmitic Acid:SD-30:NaCl 49:21:5:23:2 |
| 74* | DPPC:POPG-Na:Palmitic Acid: Albumin*:SD-30:NaCl 49:21:5:3:20:2 |
| 75 | DPPC:POPG-Na:Palmitic Acid:SP-B:SD-30:NaCl (49:21:5:3:20:2) |
| 76 | DPPC:POPG-Na:POPC:SD-30:NaCl (49:21:7:21:2) |
| 77 | DPPC:POPG:POPC:SP-B:SD-30:NaCl (49:21:7:3:18:2) |
| 78 | DPPC:POPG: POPC: Albumin*:SD-30:NaCl (49:21:7:3:18:2) |
| 79 | DPPC:POPG:Lactose:NaCl (56:24:18:2) |
| 80 | DPPC:POPG:Trehalose dihydrate: NaCl (56:24:18:2) |
| 81 | DPPC:POPG:Maltitol:NaCl (56:24:18:2) |

(continued)

| Formulation # | Description |
|---|---|
| 82 | DPPC:POPG:SP-B:Lactose:NaCl (56:24:3:15:2) |
| 83 | DPPC:POPG:SP-B: Trehalose dihydrate:NaCl (56:24:3:15:2) |
| 84 | DPPC:POPG:SP-B: Maltitol:NaCl (56:24:3:15:2) |
| 85 | DPPC:POPG-Na:SD-30:NaCl (55:20:23:2) |
| 86 | DPPC:POPG-Na: Albumin: SD-30:NaCl (55:20:3:20:2) |
| 87 | DPPC:POPG-Na:SD-30:NaCl (55:25:18:2) |
| 88 | DPPC:POPG-Na:Albumin:SD-30:NaCl (55:25:3:15:2) |
| 89 | DPPC:POPG:Raffinose:NaCl (56:24:18:2) |
| 90 | DPPC:POPG:Erithritol:NaCl (56:24:18:2) |
| 91 | DPPC:POPG:Xylitol:NaCl (56:24:18:2) |
| 92 | DPPC:POPG:Sorbitol:NaCl (56:24:18:2) |
| 93 | DPPC:POPG:Mannitol:NaCl (56:24:18:2) |
| 94 | DPPC:POPG:Maltotritol:NaCl (56:24:18:2) |
| 95 | DPPC:POPG:Sinapultide:Lactose:NaCl (56:24:3:15:2) |
| 96 | DPPC:POPG: Sinapultide: Trehalose dihydrate:NaCl (56:24:3:15:2) |
| 97 | DPPC:POPG: Sinapultide: Maltitol:NaCl (56:24:3:15:2) |
| 98 | DPPC:POPG:Sinapultide: Raffinose:NaCl (56:24:3:15:2) |
| 99 | DPPC:POPG:Sinapultide: Erithritol:NaCl (56:24:3:15:2) |
| 100 | DPPC:POPG:Sinapultide:Xylitol:NaCl (56:24:3:15:2) |
| 101 | DPPC:POPG:Sinapultide:Sorbitol:NaCl (56:24:3:15:2) |
| 102 | DPPC:POPG:Sinapultide: Mannitol:NaCl (56:24:3:15:2) |
| 103 | DPPC:POPG:Sinapultide: Maltotritol:NaCl (56:24:3:15:2) |
| 104 | DPPC:POPG-Na:SD-30:Sinapultide:NaCl (49:21:25:3:2) |
| 105 | DPPC:POPG-Na:Palmitic Acid:Sinapultide:SD-30:NaCl (49:21:5:3:20:2) |
| 106 | DPPC:POPG:POPC:Sinapultide:SD-30:NaCl (49:21:7:3:18:2) |
| 107 | DPPC:POPG: SP-B: Maltitol:NaCl (56:24:3:15:2) |
| 108 | DPPC:POPG:SP-B: Raffinose:NaCl (56:24:3:15:2) |
| 109 | DPPC:POPG:SP-B: Erithritol:NaCl (56:24:3:15:2) |
| 110 | DPPC:POPG:SP-B:Xylitol:NaCl (56:24:3:15:2) |
| 111 | DPPC:POPG:SP-B: Mannitol:NaCl (56:24:3:15:2) |
| 112 | DPPC:POPG:SP-B: Maltotritol:NaCl (56:24:3:15:2) |
| *Albumin is preferably replaced with a surfactant protein, e.g., SEQ ID No. 9 or sinapultide, in these formulations. | |

Preferably, the surfactant formulations are selected from Formulations 1-45 and 65-72 and 73-77. Preferably the surfactant formulations are selected from Formulations 1-27 and 65-72 and 73-77. Preferably Formulations 1-45 and 65-71 and 72, 73, 74, 76 and 77 (further include a surfactant protein in addition to the named components, or instead of one or more of the named components, for example as shown in Formulas 72, 75 and 77. Preferably, the surfactant protein is present in an amount of about 5 weight percent or less of the particle. Preferably the surfactant protein is derived from SP-B including but not limited to SEQ ID NOS: 1-21. Preferably the surfactant protein comprises SEQ ID NO: 9 or a homologous amino acid sequence with at least about 70%, preferably at least about 80%, preferably at least about 85%, preferably at least about 90%, or preferably at least about 95% sequence identity at the amino acid level.

A preferred surfactant formulation comprising a surfactant protein is DPPC:POPG-Na:SD-30:*surfactant pro-*

*tein*:NaCL (49:21:25:3:2). Preferably the surfactant protein is SP-B including but not limited to SEQ ID NOS: 1-21. Preferably the surfactant protein comprises SEQ ID NO: 9 or a homologous amino acid sequence with at least about 70%, preferably at least about 80%, preferably at least about 85%, preferably at least about 90%, or preferably at least about 95% sequence identity at the amino acid level. Another preferred surfactant protein is sinapultide. A preferred surfactant formulation comprising a surfactant protein in accordance with the invention is Formula 72.

Preferably, the percentage of excipient when present in the particle is lowered or eliminated in order to accommodate the additional presence of surfactant protein. Preferably in those formulations comprising albumin (Formulations 21-27, 45 and 71) at least one surfactant protein, preferably SP-B or an active fragment thereof that contains at least a portion of a lipid associating region (e.g., SEQ ID NOS: 1-21), replaces the 5% by weight albumin in the formulation. Alternatively, the ratio of the excipients can be maintained and the surfactant protein added thereto. Preferably less than about 25% by weight, preferably less than about 15% by weight, preferably less than about 10% by weight, and/or less than or equal to about 5% by weight of a surfactant protein is present in a formulation of the invention. Preferred ranges of surfactant protein include 1 to 10% by weight and about 2 to about 7% by weight. Preferably the particles of the invention comprise about 3%, 4%, 5%, 6% or 7% by weight of surfactant protein. Other surfactant containing formulations (not according to the claims) are shown in Table D:

Table D

| Formulation # | Description |
|---|---|
| 38 | DPPC:POPC (70:30) |
| 39 | DPPC:Albumin* (95:5) |
| 44 | DPPC:POPC (70:30) |
| 46 | DPPC:DOPC:Leu:NaCl (60:20:18:2) |
| 47 | DPPC:DOPC (95:5) |
| 48 | DPPC:DOPC:Leucine:NaCl (60:20:18:2) |
| 49 | DPPC:POPG:Albumin*:NaCl (48:45:5:2) |
| 50 | DPPC:DPPE:POPG:NaCl (68:10:20:2) |
| 51 | DPPC:DPPG:POPG:NaCl (68:10:20:2) |
| 52 | DPPC:SD-30:NaCl (40:58:2) |
| 53 | DPPC:SD-30:NaCl (20:78:2) |
| 54 | DPPC: L-leu:NaCl (40:58:2) |
| 55 | DPPC: L-leu:NaCl (20:78:2) |
| 56 | DPPC:SD-30:0regon Green Dye: NaCl (80:17.5:0.5:2) |
| 57 | DPPC:SD-30:Fluorescein Dye (FD):NaCl (80:17.5:0.5:2) |
| 58 | DPPC:SD-30:FD:NaCl (80:16:2:2) |
| 59 | DPPC:POPC:POPG-Na:SD-30:FD:NaCl (42:14:14:27.5:0.5:2) |
| 60 | DPPC:POPC:POPG-Na:SD-30:FD:NaCl (40:24:16:17.5:0.5:2) |
| 61 | DPPC:DOPC:SD-30 (85:5:10) |
| 62 | DPPC:DPPG:POPG-Na:Albumin*) (60:10:25:5) |
| 63 | DPPC:POPG-Na (70:30) |
| 64 | DPPC:POPG-Na:NaCl (68:30:2) |
| *Albumin is preferably replaced with a surfactant protein in these formulations ||

Methods for Preparing Dry Powders and Dry Particles

The respirable dry particles and dry powders can be prepared using any suitable method. Many suitable methods for preparing respirable dry powders and particles are conventional in the art, and include single and double emulsion solvent evaporation, spray drying, milling (e.g., jet milling), blending, solvent extraction, solvent evaporation, phase separation, simple and complex coacervation, interfacial polymerization, suitable methods that involve the use of supercritical carbon dioxide ($CO_2$), and other suitable methods. Respirable dry particles can be made using methods for making microspheres or microcapsules known in the art. These methods can be employed under conditions that result in the formation of respirable dry particles with desired aerodynamic properties (e.g., aerodynamic diameter and geometric diameter). If desired, respirable dry particles with desired properties, such as size and density, can be selected using suitable methods, such as sieving.

The respirable dry particles are preferably spray dried. Suitable spray-drying techniques are described, for example, by K. Masters in "Spray Drying Handbook", John Wiley & Sons, New York (1984). Generally, during spray-drying, heat from a hot gas such as heated air or nitrogen is used to evaporate a solvent from droplets formed by atomizing a continuous liquid feed. If desired, the spray drying or other instruments, e.g., jet milling instrument, used to prepare the dry particles can include an inline geometric particle sizer that determines a geometric diameter of the respirable dry particles as they are being produced, and/or an inline aerodynamic particle sizer that determines the aerodynamic diameter of the respirable dry particles as they are being produced.

For spray drying, solutions, emulsions or suspensions that contain the components of the dry particles to be produced in a suitable solvent (e.g., aqueous solvent, organic solvent, aqueous-organic mixture or emulsion) are distributed to a drying vessel via an atomization device. For example, a nozzle or a rotary atomizer may be used to distribute the solution or suspension to the drying vessel. Examples of suitable spray dryers that can be outfitted with either a rotary atomizer or a nozzle, include, Mobile Minor Spray Dryer or the Model PSD-1, both manufactured by Niro, Inc. (Denmark). Actual spray drying conditions will vary depending, in part, on the composition of the spray drying solution or suspension and material flow rates.

The person of ordinary skill will be able to determine appropriate conditions based on the compositions of the solution, emulsion or suspension to be spray dried, the desired particle properties and other factors. In general, the inlet temperature to the spray dryer is about 50° C to about 200° C, and preferably is about 60° C to about 150° C. The spray dryer outlet temperature will vary depending upon such factors as the feed temperature and the properties of the materials being dried. Generally, the outlet temperature is about 40° C to about 150° C, preferably about 50° C to about 120° C, or about 60° C to about 90° C. If desired, the respirable dry particles that are produced can be fractionated by volumetric size, for example, using a sieve, or fractioned by aerodynamic size, for example, using a cyclone, and/or further separated according to density using techniques known to those of skill in the art.

To prepare the respirable dry particles of the invention, generally, a solution, emulsions or suspension that contains the desired components of the dry powder (i.e., a feed stock) is prepared and spray dried under suitable conditions. Preferably, the dissolved or suspended solids concentration in the feed stock is at least about 1 g/L, at least about 2 g/L, at least about 5 g/L, at least about 10 g/L, at least about 15 g/L, at least about 20 g/L, at least about 30 g/L, at least about 40 g/L, at least about 50 g/L, at least about 60 g/L, at least about 70 g/L, at least about 80 g/L, at least about 90 g/L, or at least about 100 g/L. The feed stock can be provided by preparing a single solution or suspension by dissolving or suspending suitable components (e.g., salts, excipients, other active ingredients) in a suitable solvent. The solvent, emulsion or suspension can be prepared using any suitable methods, such as bulk mixing of dry and/or liquid components or static mixing of liquid components to form a combination. For example, a hydrophilic component (e.g., an aqueous solution) and a hydrophobic component (e.g., an organic solution) can be combined using a static mixer to form a combination. The combination can then be atomized to produce droplets, which are dried to form respirable dry particles. Preferably, the atomizing step is performed immediately after the components are combined in the static mixer.

The feed stock, or components of the feed stock, can be prepared using any suitable solvent, such as an organic solvent, an aqueous solvent or mixtures thereof. Suitable organic solvents that can be employed include but are not limited to alcohols such as, for example ethanol, methanol, propanol, isopropanol, butanols, and others. Other organic solvents include but are not limited to perfluorocarbons, dichloromethane, chloroform, ether, ethyl acetate, methyl tert-butyl ether and others. Co-solvents that can be employed include an aqueous solvent and an organic solvent, such as, but not limited to, the organic solvents as described above. Aqueous solvents include water and buffered solutions.

The feed stock or components of the feed stock can have any desired pH, viscosity or other properties. If desired, a pH buffer can be added to the solvent or co-solvent or to the formed mixture. Generally, the pH of the mixture ranges from about 3 to about 8.

Respirable dry particles and dry powders can be fabricated and then separated, for example, by filtration or centrifugation by means of a cyclone, to provide a particle sample with a preselected size distribution. For example, greater than about 30%, greater than about 40%, greater than about 50%, greater than about 60%, greater than about 70%, greater than about 80%, or greater than about 90% of the respirable dry particles in a sample can have a diameter within a selected range. The selected range within which a certain percentage of the respirable dry particles fall can be, for example, any of the size ranges described herein, such as between about 0.1 to about 3 microns VMGD.

The diameter of the respirable dry particles, for example, their VMGD, can be measured using an electrical zone sensing instrument such as a Multisizer Iie, (Coulter Electronic, Luton, Beds, England), or a laser diffraction instrument such as a HELOS system (Sympatec, Princeton, N.J.). Other instruments for measuring particle geometric diameter are well known in the art. The diameter of respirable dry particles in a sample will range depending upon factors such as particle composition and methods of synthesis. The distribution of size of respirable dry particles in a sample can be selected to permit optimal deposition within targeted sites within the respiratory system.

Experimentally, aerodynamic diameter can be determined using time of flight (TOF) measurements. For example, an instrument such as the Model 3225 Aerosizer DSP Particle Size Analyzer (Amherst Process Instrument, Inc., Amherst, Mass.) can be used to measure aerodynamic diameter. The Aerosizer measures the time taken for individual respirable dry particles to pass between two fixed laser beams.

Aerodynamic diameter also can be experimentally determined directly using conventional gravitational settling methods, in which the time required for a sample of respirable dry particles to settle a certain distance is measured. Indirect methods for measuring the mass median aerodynamic diameter include the Andersen Cascade Impactor and the multistage liquid impinger (MSLI) methods. The methods and instruments for measuring particle aerodynamic diameter are well known in the art.

Tap density is a measure of the envelope mass density characterizing a particle. The envelope mass density of a particle of a statistically isotropic shape is defined as the mass of the particle divided by the minimum sphere envelope volume within which it can be enclosed. Features which can contribute to low tap density include irregular surface texture and porous structure. Tap density can be measured by using instruments known to those skilled in the art such as the Dual Platform Microprocessor Controlled Tap Density Tester (Vankel, N.C.), a GEOPYC™ instrument (Micrometrics Instrument Corp., Norcross, Ga.), or SOTAX Tap Density Tester model TD2 (SOTAX Corp., Horsham, Pa.). Tap density can be determined using the method of USP Bulk Density and Tapped Density, United States Pharmacopia convention, Rockville, Md., 10th Supplement, 4950-4951, 1999.

Fine particle fraction can be used as one way to characterize the aerosol performance of a dispersed powder. Fine particle fraction describes the size distribution of airborne respirable dry particles. Gravimetric analysis, using a Cascade impactor, is one method of measuring the size distribution, or fine particle fraction, of airborne respirable dry particles. The Andersen Cascade Impactor (ACI) is an eight-stage impactor that can separate aerosols into nine distinct fractions based on aerodynamic size. The size cutoffs of each stage are dependent upon the flow rate at which the ACI is operated. The ACI is made up of multiple stages consisting of a series of nozzles (i.e., a jet plate) and an impaction surface (i.e., an impaction disc). At each stage an aerosol stream passes through the nozzles and impinges upon the surface. Respirable dry particles in the aerosol stream with a large enough inertia will impact upon the plate. Smaller respirable dry particles that do not have enough inertia to impact on the plate will remain in the aerosol stream and be carried to the next stage. Each successive stage of the ACI has a higher aerosol velocity in the nozzles so that smaller respirable dry particles can be collected at each successive stage.

If desired, a two- or three-stage collapsed ACI can also be used to measure fine particle fraction. The two-stage collapsed ACI consists of only the top stage (S0) and the filter stage of the eight-stage ACI and allows for the collection of two separate powder fractions. Specifically, a two-stage collapsed ACI is calibrated so that the fraction of powder that is collected on S0 is composed of non-respirable dry particles that have an aerodynamic diameter of greater than 5.6 microns. The fraction of powder passing S0 and depositing on the filter stage is thus composed of respirable dry particles having an aerodynamic diameter of less than 5.6 microns. The airflow at such a calibration is approximately 60 L/min. Similarly, the three-stage collapsed ACI consists of collection stage S0, S2 and the filter stage and provides fractions of powder of an aerodynamic diameter greater than 5.6 microns, less than 5.6 microns and less than 3.4 microns. The FPF (<5.6) has been demonstrated to correlate to the fraction of the powder that is able to make it into the lung of the patient, while the FPF(<3.4) has been demonstrated to correlate to the fraction of the powder that reaches the deep lung of a patient. These correlations provide a quantitative indicator that can be used for particle optimization.

An ACI can be used to approximate the emitted dose, which herein is called gravimetric recovered dose and analytical recovered dose. "Gravimetric recovered dose" is defined as the ratio of the powder weighed on all stage filters of the ACI to the nominal dose. "Analytical recovered dose" is defined as the ratio of the powder recovered from rinsing all stages, all stage filters, and the induction port of the ACI to the nominal dose. The FPF_TD(<5.0) is the ratio of the interpolated amount of powder depositing below 5.0 μm on the ACI to the nominal dose. The FPF_RD(<5.0) is the ratio of the interpolated amount of powder depositing below 5.0 μm on the ACI to either the gravimetric recovered dose or the analytical recovered dose.

Another way to approximate emitted dose is to determine how much powder leaves its container, e.g., capture or blister, upon actuation of a dry powder inhaler (DPI). This takes into account the percentage leaving the capsule, but does not take into account any powder depositing on the DPI. The emitted dose is the ratio of the weight of the capsule with the dose before inhaler actuation to the weight of the capsule after inhaler actuation. This measurement can also be called the capsule emitted powder mass (CEPM).

A Multi-Stage Liquid Impinger (MSLI) is another device that can be used to measure fine particle fraction. The Multi-stage liquid Impinger operates on the same principles as the ACI, although instead of eight stages, MSLI has five. Additionally, each MSLI stage consists of an ethanol-wetted glass frit instead of a solid plate. The wetted stage is used to prevent particle bounce and re-entrainment, which can occur when using the ACI.

## EXAMPLES

The present invention will be better understood in connection with the following Examples. However, it should be understood that these examples are for illustrative purposes only and are not meant to limit the scope of the invention.

## Example 1- Materials and Methods

### ABBREVIATIONS:

DOPC: 1,2-di-(9Z-octadecenoyl)-*sn*-glycero-3-phosphocholine
DPPC: Dipalmitoylphosphatidylcholine
DPPE: 1,2-dihexadecanoyl-sn-glycero-3-phosphoethanolamine
DPPG-Na: 1,2-Dipalmitoyl-sn-glycero-3-phosphoglycerol, sodium salt
gPSD: Average geometric particle size (d50)
L-leu or Leu: Leucine
Mg Lactate: Magnesium lactate trihydrate
PA: palmitic acid
n.c.: Not calculated
n.d.: Not detected
POPC: 1-palmitoyl-2-oleoyl-*sn*-glycero-3-phosphocholine
POPE: 1-palmitoyl-2-oleoyl-*sn*-glycero-3-phosphoethanolamine
POPG-Na or POPG: 1-palmitoyl-2-oleoyl-*sn*-glycero-3-phospho-(1'-*rac*-glycerol) (sodium salt)
SC-D: Semicrystalline due to lipid bilayer
SC-DE: Semicrystalline due to lipid bilayer and excipients
SD-30: Polyglycitol
TGA-120: Total weight loss or volatiles from heating a sample up to 120 °C at a heating ramp of 20 °C/ min
Um or $\mu$m: micrometer
XRD: X-Ray powder diffraction

Formulation numbers that include a dash (e.g. Formula 31-1, Formula 31-2 and so on) indicate that the components of the formula and the ratios of the components of the formula are identical to those of the parent formula (e.g. Formula 31), however, formulations containing dashes (e.g. Formula 31-1) are not from the same lot as the parent formulation and may also have been processed using different process parameters as compared to the parent formulation lot.

### REAGENTS:

1. Polyglycitol Stabilite SD-30 (Grain Processing Corp., Muscatine, IA, USA)
2. L-leucine (Ajinomoto Aminosciences LLC, Raleigh, NC, USA)
3. DPPC (Lipoid GmbH, Steinhausen, Switzerland)
4. POPG-Na (Avanti Polar Lipids, Alabaster, AL, USA)
5. POPC (Lipoid GmbH, Steinhausen, Switzerland)
6. Sodium chloride (VWR, Radnor, PA, USA)
7. Ethanol, 200 Proof (Pharmco Products Inc, Brookfield, CT, USA)
8. Civitas Purified Water
9. DOPC
10. DPPE
11. DPPG-Na
12. Albumin
13. Mg Lactate
14. Lactose monohydrate (Sigma, St. Louis, MO, USA)
15. Trehalose dihydrate (Sigma, St. Louis, MO, USA)
16. Maltitol (Sigma, St. Louis, MO, USA)

**EQUIPMENT:**

1. GEA Niro PSD, Size 1 (GEA Process Engineering, Soborg, Denmark).
2. Glove box equipped with N2 supply and accordion gloves (Bel-art products, Wayne, NJ).
3. Buchi B-290 spray dryer.

**ANALYSIS:**

Selected formulations were tested for the following parameters:

1. gPSD (Average geometric particle size in microns (d50));
2. Fine particle fraction of the total dose < 5.6 um and/or 3.4 um (grav.);
3. XRPD (X-ray powder diffraction);
4. Low T1 (Characteristic temperature(s) of the first thermal event(s) observed during a DSC scan at 20 °C/min);
5. Low T2 (Characteristic temperature(s) of the second set of thermal event(s) observed during a DSC scan at 20 °C/min); and
6. TGA-120 (%) (Volatiles loss by 120 °C).

**Example 2-Spray Drying Process Parameters.**

The powders were made using Niro PSD-1 with the process parameters listed here.

| Process Parameters | Value |
|---|---|
| Total solid concentration (g/L) | 4 |
| Inlet temperature (°C) | 63 |
| Outlet temperature (°C) | 40 |
| Drying gas flow rate (kg/hr) | 125 |
| Atomization gas flow rate (g/min) | 40 |
| Aqueous flow (mL/min) | 10 |
| Organic flow (mL/min) | 10 |
| Liquid skid heat exchanger (°C) | 55 |
| Water feed temperature (°C) | 20 |
| Ethanol feed temperature (°C) | 55 |
| Atomization tower heat exchanger (°C) | 55 |
| Baghouse purge rate (scfh) | 20 |

**EXAMPLE 3-Formulation Development.** (not according to the claims)

Accelerated stability testing was performed on formulations comparing stability with and without the storage in the presence of 1 g silica gel. All formulations were prepared as described herein.

| Description | Desiccant | Condition | | gPSD (um) | Size 00 FPF <5.6um (%) | Size 00 FPF <3.4 um (%) | XRPD | TG-A-120 (%) | DSC Low T1 (°C) | DSC Low T2 (°C) |
|---|---|---|---|---|---|---|---|---|---|---|
| DPPC:POPG-Na:SD-30:NaCl (55:20:23:2) | NA | t=0 | | 6.7 | 70 | 57 | SC-D | 1.39 | 44.4 | 64 |
| | No | 40 °C | 2 wk | | 58 | 39 | SC-D | 1.48 | 42.3 | 64.2 |
| | | | 1 mth | | 58 | 43 | SC-D | 1.47 | 42.4 | 64.4 |
| | | | 3 mth | | 55 | 39 | | | | |
| | Yes | 40 °C | 2 wk | | 67 | 52 | SC-D | 0.67 | n.c. | 65.6 |
| | | | 1 mth | | 60 | 48 | SC-D | 0.73 | n.c. | 65.3 |
| | | | 3 mth | | 73 | 67 | | | | |
| DPPC:POPG-Na:Albumin:SD-30:NaCl (55:20:3:20:2) | NA | t=0 | | 5.4 | 60 | 46 | SC-D | 1.57 | 45.3 | 63.7 |
| | No | 40 °C | 2 wk | | 69 | 49 | SC-D | 1.59 | 42.4 | 63.7 |
| | | | 1 mth | | 41 | 29 | SC-D | 1.58 | 42.6 | 64.2 |
| | | | 3 mth | | 66 | 47 | | | | |
| | Yes | 40 °C | 2 wk | | 74 | 62 | SC-D | 0.96 | n.c. | 65.4 |
| | | | 1 mth | | 55 | 41 | SC-D | 0.75 | n.c. | 64.8 |
| | | | 3 mth | | 77 | 71 | | | | |

| Description | Desiccant | Condition | | gPSD (um) | Size 00 FPF <5.6um (%) | Size 00 FPF <3.4 um (%) | XRPD | TG-A-120 (%) | DSC Low T1 (°C) | DSC Low T2 (°C) |
|---|---|---|---|---|---|---|---|---|---|---|
| DPPC:POPG-Na:SD-30:NaCl (55:25:18:2) | NA | t=0 | | 5.8 | 60 | 46 | SC-D | 1.47 | 41.9 | 64.2 |
| | No | 40 °C | 2 wk | | 27 | 19 | SC-D | 2.00 | 30.9 | 63 |
| | | | 1 mth | | 24 | 17 | SC-D | 2.12 | 30.3 | 62.8 |
| | | | 3 mth | | 26 | 16 | | | | |
| | Yes | t=2wk40C | | | 56 | 36 | SC-D | 0.68 | n.c. | 65.5 |
| | | 40 °C | 1mnth | | 53 | 36 | SC-D | 0.67 | n.c. | 64.9 |
| | | | 3mnth | | 75 | 53 | | | | |
| DPPC:POPG-Na:Albumin:SD-30:NaCl (55:25:3:15:2) | NA | t=0 | | 7.2 | 62 | 49 | SC-D | 1.20 | 44.9 | 63.3 |
| | No | t=2wk40C | | | 43 | 29 | SC-D | 1.99 | 34.6 | 64.3 |
| | | 40 °C | 1 mnth | | 3 | 2 | SC-D | 1.66 | 40.3 | 64.4 |
| | | | 3mnth | | 43 | 24 | | | | |
| | Yes | 40 °C | 2 wk | | 65 | 46 | SC-D | 0.73 | n.c. | 65.1 |
| | | | 1mnth | | 61 | 46 | SC-D | 0.55 | n.c. | 65.8 |
| | | | 3mnth | | 71 | 54 | | | | |

| Description | Desiccant | Condition | | gPSD (um) | Size 00 FPF <5.6um (%) | Size 00 FPF <3.4 um (%) | XRPD | TG-A-120 (%) | DSC | |
|---|---|---|---|---|---|---|---|---|---|---|
| | | | | | | | | | Low T1 (°C)* | Low T2 (°C) |
| DPPC:POPG-Na:Leu:NaCl (49:21:28:2) | NA | t=0 | | 4.6 | 57 | 39 | SC-DE | 1.36 | 45.3 | 66.1, 75.6, 126 |
| | No | 40 °C | 2 wk | | 33 | 24 | SC-DE | 1.64 | 47.1 | 61, 66.9, 75.8, 128.8 |
| | | | 1 mth | | 16 | 13 | SC-DE | 1.73 | 46.5 | 60.8, 66.6, 75.7, 128.2 |
| | | | 3 mth | | 22 | 15 | | | | |
| | Yes | 40 °C | 2 wk | | 53 | 36 | SC-DE | 0.75 | 50.2 | 63.1, 70.3, 80.6, 125.4 |
| | | | 1 mth | | 56 | 38 | SC-DE | 0.66 | 47.9 | 61.9, 69.9, 79.2, 122 |
| | | | 3 mth | | 60 | 39 | | | | |

*Low T1: Low energy transition- minor event transition may be artificial due to baseline noise

| Description | Desiccant | Condition | | gPSD (um) | Size 00 FPF <5.6um (%) | Size 00 FPF <3.4 um (%) | XRPD | TG-A-120 (%) | DSC | |
|---|---|---|---|---|---|---|---|---|---|---|
| | | | | | | | | | Low T1 (°C) | Low T2 (°C) |
| DPPC:POPG-Na:Leu:NaCl (56:24:18:2) | NA | t=0 | | 5.9 | 42 | 29 | SC-DE | 1.47 | 45.5 | 65.9, 75.5, 126.3 |
| | No | 40 °C | 2 wk | | 16 | 13 | SC-DE | 1.92 | 46.7 | 61.1, 66.9, 76.2, 128.1, 155.7 |
| | | | 1 mth | | 23 | 20 | SC-DE | 1.66 | 46 | 60.7, 66.7, 78, 127.8 |
| | | | 3 mth | | 14 | 12 | | | | |
| | Yes | 40 °C | 2 wk | | 48 | 35 | SC-DE | 0.74 | 36.1 | 62.1, 69.8, 79.5, 124.2 |
| | | | 1 mth | | 48 | 35 | SC-DE | 0.84 | 40.3 | 62.7, 70.8, 80.6, 125.7 |
| | | | 3 mth | | 50 | 37 | | | | |

| Description | # 0.25 g desiccants | Condition | | gPSD (um) | Size 00 FPF <5.6um (%) | Size 00 FPF <3.4 um (%) | XRPD | TG-A-120 (%) | DSC Low T1 (°C) | DSC Low T2 (°C) |
|---|---|---|---|---|---|---|---|---|---|---|
| DPPC:POPG-Na:Albumin:SD-30:NaCl (55:20:3:20:2) | 0 | t=0 | | 6.7 | 60 | 46 | SC-D | 1.57 | 45.3 | 63.7 |
| DPPC:POPG-Na:Albumin:SD-30:NaCl (55:20:3:20:2) plus 1 g of silica gel in pkg | 4 | 40 °C | 2 wk | | 77 | 68 | SC-D | 0.92 | n.c. | 64.2 |
| | | | 1 mth | | 69 | 62 | SC-D | 0.75 | | 64.9 |
| DPPC:POPG-Na:Albumin:SD-30:NaCl (55:20:3:20:2) plus 2 g of silica gel in pkg | 8 | 40 °C | 2 wk | | 72 | 65 | SC-D | 0.78 | n.c. | 65.6 |
| | | | 1 mth | | 79 | 67 | SC-D | 0.69 | n.c. | 65.5 |

| Description | Desiccant | Condition | | gPSD (um) | Size 00 FPF <5.6um (%) | Size 00 FPF <3.4 um (%) | XRPD | TG-A-120 (%) | DSC Low T1 (°C) | DSC Low T2 (°C) |
|---|---|---|---|---|---|---|---|---|---|---|
| DPPC:POPG:Lactose:NaCl (56:24:18:2) | NA | t=0 | | 5 | 72 | 61 | SC-D | 1.54 | 42.5 | 60.3 |
| | No | 40 °C | 2 wk | | 20 | 16 | SC-D | 1.83 | 40.9 | 60.3 |
| | | | 1 mth | | 15 | 9 | SC-D | 1.9 | 39.4 | 58.5 |
| | Yes | 40 °C | 2 wk | | 79 | 70 | SC-D | 0.61 | n.c. | 62.8 |
| | | | 1 mth | | 82 | 69 | SC-D | 0.53 | n.c. | 63.3 |

| Description | Desiccant | Condition | | gPSD (um) | Size 00 FPF <5.6um (%) | Size 00 FPF <3.4 um (%) | XRPD | TG-A-120 (%) | DSC Low T1 (°C) | DSC Low T2 (°C) |
|---|---|---|---|---|---|---|---|---|---|---|
| *DPPC:POPG:Trehalose dihydrate: NaCl (56:24:18:2)* | *NA* | *t-0* | | *3.2* | *80* | *56* | *SC-D* | *1.17* | *43.9* | *59.6* |
| | No | 40 °C | 2 wk | | 12 | 9 | SC-D | 1.85 | 38 | 58.3 |
| | | | 1 mth | | 18 | 15 | SC-D | 2.03 | 35.1 | 57.6 |
| | Yes | 40 °C | 2 wk | | 77 | 52 | SC-D | 0.58 | n.c. | 63.1 |
| | | | 1 mth | | 79 | 61 | SC-D | 0.48 | n.c. | 61.7 |

| Description | Desiccant | Condition | | gPSD (um) | Size 00 FPF <5.6um (%) | Size 00 FPF <3.4 um (%) | XRPD | TG-A-120 (%) | DSC Low T1 (°C) | DSC Low T2 (°C) |
|---|---|---|---|---|---|---|---|---|---|---|
| | NA | t=0 | | 4.8 | 65 | 52 | SC-D | 1.18 | 28.7 | 53 |
| DPPC:POPG:Maltitol:NaCl (56:24:18:2) | No | 40 °C/ 75% RH | 2 wk | | 5 | 4 | | | | |
| | Yes | 40 °C/ 75% RH | 2 wk | | 22 | 18 | | | | |

## Claims

1. A container comprising:

   (a) a capsule containing a respirable, dry powder particle surfactant formulation for pulmonary delivery comprising:

   i) at least 30% DPPC by weight of the particle;
   ii) 0.1 to 3% NaCl by weight of the particle;
   iii) 1% to 10% by weight of the particle of a surfactant protein selected from the group consisting of: SP-A, SP-B, SP-C and SP-D, or an active fragment thereof that contains at least a portion of a lipid associating region; and
   iv) 10% to 30% by weight of an excipient selected from the group consisting of any one or more of leucine, trehalose, lactose, mannitol, sugar alcohol, or a hydrogenated starch hydrolysate (HSH);

   wherein all components of the dry powder particles amount to 100 weight percent; and
   (b) a desiccant.

2. The container of claim 1, wherein the desiccant is a silica gel.

3. The container of claim 1 or 2, wherein the desiccant is packaged in a sachet.

4. The container of any one of claims 1-3, wherein the formulation further comprises at least one or more of DOPC, POPC, DPPE, DPPG or POPG.

5. The container of any one of the preceding claims, wherein the formulation comprises 1% to 10% by weight of the particle of a surfactant protein selected from the group consisting of: SEQ ID NOS: 1-21 or an amino acid sequence homologous thereto with at least 90% identity at the amino acid level.

6. The container of any one of the preceding claims, wherein the formulation further comprises 1% to 10% by weight of the particle of sinapultide.


## Patentansprüche

1. Behälter, umfassend:

   (a) eine Kapsel mit einer lungengängigen Trockenpulverpartikel-Tensidformulierung zur pulmonalen Zuführung, die Folgendes umfasst:

i) mindestens 30 Gew.-%, bezogen auf das Partikel, DPPC;

ii) 0,1 bis 3 Gew.-%, bezogen auf das Partikel, NaCl;

iii) 1 bis 10 Gew.-%, bezogen auf das Partikel, eines Tensidproteins, das aus der Gruppe bestehend aus SP-A, SP-B, SP-C und SP-D bzw. einem aktiven Fragment davon, das mindestens einen Teil einer Lipidassoziationsregion enthält, ausgewählt ist, und

iv) 10 bis 30 Gew.-% eines Hilfsstoffs, der aus der Gruppe bestehend aus einem oder mehreren von Leucin, Trehalose, Lactose, Mannit, Zuckeralkohol oder einem hydrierten Stärkehydrolysat (HSH) ausgewählt ist;

wobei alle Komponenten der Trockenpulverpartikel zusammen 100 Gewichtsprozent ergeben; und

(b) ein Trockenmittel.

**2.** Behälter nach Anspruch 1, wobei es sich bei dem Trockenmittel um ein Kieselgel ist.

**3.** Behälter nach Anspruch 1 oder 2, wobei das Trockenmittel in einem Tütchen verpackt ist.

**4.** Behälter nach einem der Ansprüche 1 bis 3, wobei die Formulierung ferner mindestens eines oder mehrere von DOPC, POPC, DPPE, DPPG oder POPG umfasst.

**5.** Behälter nach einem der vorhergehenden Ansprüche, wobei die Formulierung 1 bis 10 Gew.-%, bezogen auf das Partikel, eines Tensidproteins umfasst, das aus der Gruppe bestehend aus SEQ ID NO: 1-21 oder einer dazu homologen Aminosäuresequenz mit mindestens 90 % Identität auf der Aminosäureebene ausgewählt ist.

**6.** Behälter nach einem der vorhergehenden Ansprüche, wobei die Formulierung ferner 1 bis 10 Gew.-%, bezogen auf das Partikel, Sinapultid umfasst.

## Revendications

**1.** Récipient comprenant :

(a) une capsule contenant une formulation de tensioactif de particules de poudre sèche respirable pour administration pulmonaire comprenant :

i) au moins 30 % en poids des particules de DPPC ;

ii) 0,1 % à 3 % en poids de la particule ;

iii) 1 % à 10 % en poids des particules d'une protéine tensioactive choisie dans le groupe constitué par : SP-A, SP-B, SP-C et SP-D, ou un fragment actif de celles-ci qui contient au moins une partie d'une région d'association lipidique ; et

iv) 10 % à 30 % en poids d'un excipient choisi dans le groupe constitué par l'un ou plusieurs parmi leucine, tréhalose, lactose, mannitol, alcool de sucre, ou un hydrolysat d'amidon hydrogéné (HSH) ;

dans lequel tous les composants des particules de poudre sèche représentent 100 % en poids ; et

(b) un siccatif.

**2.** Récipient selon la revendication 1, dans lequel le siccatif est un gel de silice.

**3.** Récipient selon la revendication 1 ou 2, dans lequel le siccatif est emballé dans un sachet.

**4.** Récipient selon l'une des revendications 1 à 3, comprenant en outre au moins un ou plusieurs parmi DOPC, POPC, DPPE, DPPG ou POPG.

**5.** Récipient selon l'une des revendications précédentes, la formulation comprenant 1 % à 10 % en poids des particules d'une protéine tensioactive choisie dans le groupe constitué par : SEQ ID NO: 1-21 ou une séquence d'acides aminés homologue à celle-ci ayant au moins 90 % d'identité au niveau des acides aminés.

**6.** Récipient selon l'une quelconque des revendications précédentes, dans lequel la formulation comprend en outre 1 % à 10 % en poids de la particule de sinapultide.

**FIG. 1**

**FIG. 2**

Sample: 429070 t=0 17Oct17
Size: 3.1150 mg
Method: Ramp
Comment: DSC pan

TGA

File: P:...\G26\TGA\429070 t=0 17Oct17.001
Operator: HC
Run Date: 17-Oct-2017 08:19
Instrument: TGA Q50 V20.13 Build 39

1.537%
(0.04788mg)

Temperature (°C)

Universal V4.5A TA Instruments

**FIG. 3A**

Sample: 429070 t=0 17Oct17
Size: 3.1150 mg
Method: Ramp
Comment: DSC pan

TGA

File: P:...\G26\TGA\429070 t=0 17Oct17.001
Operator: HC
Run Date: 17-Oct-2017 08:19
Instrument: TGA Q50 V20.13 Build 39

1.537%
(0.04788mg)

Universal V4.5A TA Instruments

**FIG. 3B**

Sample: 429070-1g t=2wk40C 17Oct17
Size: 3.3630 mg
Method: Ramp
Comment: DSC pan

File: P:...\TGA\429070-1g t=2wk40C 17Oct17.001
Operator: HC
Run Date: 17-Oct-2017 09:24
Instrument: TGA Q50 V20.13 Build 39

TGA

0.6109%
(0.02054mg)

Weight (%)

Temperature (°C)

Universal V4.5A TA Instruments

**FIG. 3C**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62609275
- WO 9726863 A
- US 5260273 A, Cochrane
- US 8563683 B
- US 94687288 B
- US 4995385 A
- US 4069819 A

### Non-patent literature cited in the description

- USP Section 601 Aerosols, Metered-Dose Inhalers and Dry Powder Inhalers, Delivered-Dose Uniformity, Sampling the Delivered Dose from Dry Powder Inhalers. *United States Pharmacopia Convention, Rockville, Md., 13th Revision*, 2007, 222-225
- **TATUSOVA, T. A. et al.** *FEMS Microbiol Lett*, 1999, vol. 174, 187-188
- **KARLIN** ; **ALTSCHUL**. *Proc. Natl. Acad. Sci. USA*, 1990, vol. 87 (6), 2264-8
- USP Bulk Density and Tapped Density. *United States Pharmacopia convention, Rockville, Md., 10th Supplement*, 1999, 4950-4951
- **K. MASTERS**. Spray Drying Handbook. John Wiley & Sons, 1984